# EUROPEAN PATENT APPLICATION

(11) **EP 2 468 303 A1**
(43) Date of publication of application: **27.06.2012**
(21) Application number: 10195020.2
(22) Date of filing: 14.12.2010
(51) Int. Cl.: A61K 47/48, A61K 31/7115, A61K 31/712, A61P 31/18

(54) **Functionalized nucleic acids and particles comprising them for the treatment of HIV infections**

(71) Applicant: Centre National de la Recherche Scientifique, 75016 Paris (FR); Ecole Normale Superieure De Cachan, 94235 Cachan Cedex (FR)
(72) Inventor: Mouscadet, Jean-François, 92330, SCEAUX (FR); Delelis, OLivier, 78220, VIROFLAY (FR); Subra, Frédéric, 75009, PARIS (FR); Divita, Gilles, 34130, MAUGUIO (FR); Zatsepin, Timofey, 123423, MOSCOU (RU); Agapkina, Yulia, MOSCOU (RU); Gottikh, Marina, MOSCOU (RU)
(74) Representative: Vial, Lionel

(57) **Abstract**

The present invention relates to a particle comprising a functionalized nucleic acid non-covalently associated to a cell-penetrating peptide, wherein:
• the functionalized nucleic acid comprises, or consists of:
- an HIV integrase-inhibiting oligonucleotide comprising from 11 to 20 contiguous nucleotides; and
- at least one functionalizing group of the following formula (I) covalently linked to the oligonucleotide:

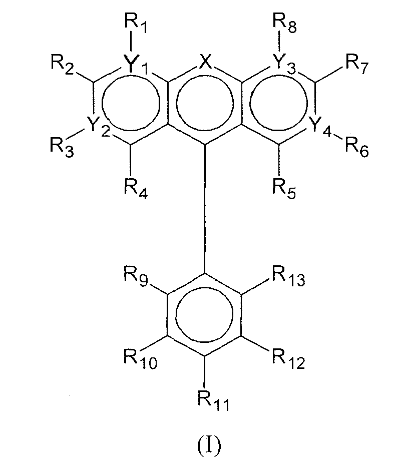

• the cell-penetrating peptide comprises from 12 to 30 contiguous amino acids;
• the particle is liable to be obtained by contacting the functionalized nucleic acid with the cell-penetrating peptide in a 1/5 to 1/15 molar ratio.

SEQUENCE LISTING

<110>  CENTRE NATIONAL DE LA RECHERCHE SCIENTIFIQUE
       ECOLE NORMALE SUPERIEURE DE CACHAN

<120>  FUNCTIONALIZED NUCLEIC ACIDS AND PARTICLES COMPRISING THEM FOR
       THE TREATMENT OF HIV INFECTIONS

<130>  F644-303 EP

<160>  7

<170>  PatentIn version 3.5

<210>  1
<211>  11
<212>  DNA
<213>  Artificial sequence

<220>
<223>  Anti-HIV integrase oligonucleotide


<220>
<221>  misc_feature
<222>  (3)..(3)
<223>  u or t

<220>
<221>  misc_feature
<222>  (4)..(4)
<223>  u or t

<220>
<221>  misc_feature
<222>  (5)..(5)
<223>  u or t

<220>
<221>  misc_feature
<222>  (6)..(6)
<223>  u or t

<220>
<221>  misc_feature
<222>  (7)..(7)
<223>  u or t

<220>
<221>  misc_feature
<222>  (9)..(9)
<223>  u or t

<220>
<221>  misc_feature
<222>  (11)..(11)
<223>  u or t

<400>  1
ggnnnnngng n                                                          11


<210>  2
<211>  11
<212>  DNA
<213>  Artificial sequence

<220>

<223>  Anti-HIV integrase oligonucleotide

<220>
<221>  misc_feature
<222>  (1)..(1)
<223>  u or t

<220>
<221>  misc_feature
<222>  (3)..(3)
<223>  u or t

<220>
<221>  misc_feature
<222>  (10)..(10)
<223>  u or t

<400>  2
ncncacaacn a                                                          11

<210>  3
<211>  11
<212>  DNA
<213>  Artificial sequence

<220>
<223>  Anti-HIV integrase oligonucleotide

<220>
<221>  misc_feature
<222>  (2)..(2)
<223>  u or t

<220>
<221>  misc_feature
<222>  (6)..(6)
<223>  u or t

<220>
<221>  misc_feature
<222>  (10)..(10)
<223>  u or t

<400>  3
cngacngcan c                                                          11

<210>  4
<211>  21
<212>  PRT
<213>  Artificial sequence

<220>
<223>  Cell-penetrating peptide

<400>  4

Lys Glu Thr Trp Phe Glu Thr Trp Phe Thr Glu Trp Ser Gln Pro Lys
1               5                   10                  15

Lys Lys Arg Lys Val
            20

<210> 5
<211> 21
<212> DNA
<213> Artificial sequence

<220>
<223> HIV integrase substrate

<400> 5
gtgtggaaaa tctctagcag t 21


<210> 6
<211> 20
<212> DNA
<213> Artificial sequence

<220>
<223> HIV integrase substrate

<400> 6
actgctagag attttcacac 20


<210> 7
<211> 9719
<212> DNA
<213> Human immunodeficiency virus type 1

<400> 7
tggaagggct aattcactcc caacgaagac aagatatcct tgatctgtgg atctaccaca 60

cacaaggcta cttccctgat tagcagaact acacaccagg gccagggatc agatatccac 120

tgacctttgg atggtgctac aagctagtac cagttgagcc agagaagtta gaagaagcca 180

acaaaggaga gaacaccagc ttgttacacc ctgtgagcct gcatggaatg gatgacccgg 240

agagagaagt gttagagtgg aggtttgaca gccgcctagc atttcatcac atggcccgag 300

agctgcatcc ggagtacttc aagaactgct gacatcgagc ttgctacaag ggactttccg 360

ctggggactt tccagggagg cgtggcctgg gcgggactgg ggagtggcga gccctcagat 420

cctgcatata agcagctgct ttttgcctgt actgggtctc tctggttaga ccagatctga 480

gcctgggagc tctctggcta actagggaac ccactgctta agcctcaata aagcttgcct 540

tgagtgcttc aagtagtgtg tgcccgtctg ttgtgtgact ctggtaacta gagatccctc 600

agaccctttt agtcagtgtg gaaaatctct agcagtggcg cccgaacagg gacctgaaag 660

cgaaagggaa accagaggag ctctctcgac gcaggactcg gcttgctgaa gcgcgcacgg 720

caagaggcga ggggcggcga ctggtgagta cgccaaaaat tttgactagc ggaggctaga 780

aggagagaga tgggtgcgag agcgtcagta ttaagcgggg gagaattaga tcgatgggaa 840

aaaattcggt taaggccagg gggaaagaaa aaatataaat taaaacatat agtatgggca 900

agcagggagc tagaacgatt cgcagttaat cctggcctgt tagaaacatc agaaggctgt 960

agacaaatac tgggacagct acaaccatcc cttcagacag gatcagaaga acttagatca 1020

ttatataata cagtagcaac cctctattgt gtgcatcaaa ggatagagat aaaagacacc 1080

aaggaagctt tagacaagat agaggaagag caaaacaaaa gtaagaaaaa agcacagcaa 1140

gcagcagctg acacaggaca cagcaatcag gtcagccaaa attaccctat agtgcagaac 1200
atccaggggc aaatggtaca tcaggccata tcacctagaa ctttaaatgc atgggtaaaa 1260
gtagtagaag agaaggcttt cagcccagaa gtgataccca tgttttcagc attatcagaa 1320
ggagccaccc cacaagattt aaacaccatg ctaaacacag tggggggaca tcaagcagcc 1380
atgcaaatgt taaaagagac catcaatgag gaagctgcag aatgggatag agtgcatcca 1440
gtgcatgcag ggcctattgc accaggccag atgagagaac caaggggaag tgacatagca 1500
ggaactacta gtacccttca ggaacaaata ggatggatga caaataatcc acctatccca 1560
gtaggagaaa tttataaaag atggataatc ctgggattaa ataaaatagt aagaatgtat 1620
agccctacca gcattctgga cataagacaa ggaccaaagg aaccctttag agactatgta 1680
gaccggttct ataaaactct aagagccgag caagcttcac aggaggtaaa aaattggatg 1740
acagaaacct tgttggtcca aaatgcgaac ccagattgta agactatttt aaaagcattg 1800
ggaccagcgg ctacactaga agaaatgatg acagcatgtc agggagtagg aggacccggc 1860
cataaggcaa gagttttggc tgaagcaatg agccaagtaa caaattcagc taccataatg 1920
atgcagagag gcaattttag gaaccaaaga aagattgtta agtgtttcaa ttgtggcaaa 1980
gaagggcaca cagccagaaa ttgcagggcc cctaggaaaa agggctgttg gaaatgtgga 2040
aaggaaggac accaaatgaa agattgtact gagagacagg ctaatttttt agggaagatc 2100
tggccttcct acaagggaag gccagggaat tttcttcaga gcagaccaga gccaacagcc 2160
ccaccagaag agagcttcag gtctggggta gagacaacaa ctccccctca gaagcaggag 2220
ccgatagaca aggaactgta tcctttaact tccctcaggt cactctttgg caacgacccc 2280
tcgtcacaat aaagataggg gggcaactaa aggaagctct attagataca ggagcagatg 2340
atacagtatt agaagaaatg agtttgccag gaagatggaa accaaaaatg atagggggaa 2400
ttggaggttt tatcaaagta agacagtatg atcagatact catagaaatc tgtggacata 2460
aagctatagg tacagtatta gtaggaccta cacctgtcaa cataattgga agaaatctgt 2520
tgactcagat tggttgcact ttaaattttc ccattagccc tattgagact gtaccagtaa 2580
aattaaagcc aggaatggat ggcccaaaag ttaaacaatg gccattgaca gaagaaaaaa 2640
taaaagcatt agtagaaatt tgtacagaga tggaaaagga agggaaaatt tcaaaaattg 2700
ggcctgaaaa tccatacaat actccagtat ttgccataaa gaaaaaagac agtactaaat 2760
ggagaaaatt agtagatttc agagaactta ataagagaac tcaagacttc tgggaagttc 2820
aattaggaat accacatccc gcagggttaa aaaagaaaaa atcagtaaca gtactggatg 2880
tgggtgatgc atatttttca gttcccttag atgaagactt caggaagtat actgcattta 2940
ccatacctag tataaacaat gagacaccag ggattagata tcagtacaat gtgcttccac 3000
agggatggaa aggatcacca gcaatattcc aaagtagcat gacaaaaatc ttagagcctt 3060
ttagaaaaca aaatccagac atagttatct atcaatacat ggatgatttg tatgtaggat 3120
ctgacttaga aatagggcag catagaacaa aaatagagga gctgagacaa catctgttga 3180

ggtggggact taccacacca gacaaaaaac atcagaaaga acctccattc ctttggatgg 3240
gttatgaact ccatcctgat aaatggacag tacagcctat agtgctgcca gaaaaagaca 3300
gctggactgt caatgacata cagaagttag tggggaaatt gaattgggca agtcagattt 3360
acccagggat taaagtaagg caattatgta aactccttag aggaaccaaa gcactaacag 3420
aagtaatacc actaacagaa gaagcagagc tagaactggc agaaaacaga gagattctaa 3480
aagaaccagt acatggagtg tattatgacc catcaaaaga cttaatagca gaaatacaga 3540
agcaggggca aggccaatgg acatatcaaa tttatcaaga gccatttaaa aatctgaaaa 3600
caggaaaata tgcaagaatg aggggtgccc acactaatga tgtaaaacaa ttaacagagg 3660
cagtgcaaaa aataaccaca gaaagcatag taatatgggg aaagactcct aaatttaaac 3720
tgcccataca aaaggaaaca tgggaaacat ggtggacaga gtattggcaa gccacctgga 3780
ttcctgagtg ggagtttgtt aatacccctc ccttagtgaa attatggtac cagttagaga 3840
aagaacccat agtaggagca gaaaccttct atgtagatgg ggcagctaac agggagacta 3900
aattaggaaa agcaggatat gttactaata gaggaagaca aaaagttgtc accctaactg 3960
acacaacaaa tcagaagact gagttacaag caatttatct agctttgcag gattcgggat 4020
tagaagtaaa catagtaaca gactcacaat atgcattagg aatcattcaa gcacaaccag 4080
atcaaagtga atcagagtta gtcaatcaaa taatagagca gttaataaaa aaggaaaagg 4140
tctatctggc atgggtacca gcacacaaag gaattggagg aaatgaacaa gtagataaat 4200
tagtcagtgc tggaatcagg aaagtactat ttttagatgg aatagataag gcccaagatg 4260
aacatgagaa atatcacagt aattggagag caatggctag tgattttaac ctgccacctg 4320
tagtagcaaa agaaatagta gccagctgtg ataaatgtca gctaaaagga gaagccatgc 4380
atggacaagt agactgtagt ccaggaatat ggcaactaga ttgtacacat ttagaaggaa 4440
aagttatcct ggtagcagtt catgtagcca gtggatatat agaagcagaa gttattccag 4500
cagaaacagg gcaggaaaca gcatattttc ttttaaaatt agcaggaaga tggccagtaa 4560
aaacaataca tactgacaat ggcagcaatt tcaccggtgc tacggttagg gccgcctgtt 4620
ggtgggcggg aatcaagcag gaatttggaa ttccctacaa tccccaaagt caaggagtag 4680
tagaatctat gaataaagaa ttaaagaaaa ttataggaca ggtaagagat caggctgaac 4740
atcttaagac agcagtacaa atggcagtat tcatccacaa ttttaaaaga aaagggggga 4800
ttggggggta cagtgcaggg gaaagaatag tagacataat agcaacagac atacaaacta 4860
aagaattaca aaaacaaatt acaaaaattc aaaattttcg ggtttattac agggacagca 4920
gaaatccact ttggaaagga ccagcaaagc tcctctggaa aggtgaaggg gcagtagtaa 4980
tacaagataa tagtgacata aaagtagtgc caagaagaaa agcaaagatc attagggatt 5040
atggaaaaca gatggcaggt gatgattgtg tggcaagtag acaggatgag gattagaaca 5100
tggaaaagtt tagtaaaaca ccatatgtat gtttcaggga aagctagggg atggttttat 5160
agacatcact atgaaagccc tcatccaaga ataagttcag aagtacacat cccactaggg 5220

gatgctagat tggtaataac aacatattgg ggtctgcata caggagaaag agactggcat 5280
ttgggtcagg gagtctccat agaatggagg aaaaagagat atagcacaca agtagaccct 5340
gaactagcag accaactaat tcatctgtat tactttgact gtttttcaga ctctgctata 5400
agaaaggcct tattaggaca catagttagc cctaggtgtg aatatcaagc aggacataac 5460
aaggtaggat ctctacaata cttggcacta gcagcattaa taacaccaaa aaagataaag 5520
ccacctttgc ctagtgttac gaaactgaca gaggatagat ggaacaagcc ccagaagacc 5580
aagggccaca gagggagcca cacaatgaat ggacactaga gcttttagag gagcttaaga 5640
atgaagctgt tagacatttt cctaggattt ggctccatgg cttagggcaa catatctatg 5700
aaacttatgg ggatacttgg gcaggagtgg aagccataat aagaattctg caacaactgc 5760
tgtttatcca ttttcagaat tgggtgtcga catagcagaa taggcgttac tcgacagagg 5820
agagcaagaa atggagccag tagatcctag actagagccc tggaagcatc caggaagtca 5880
gcctaaaact gcttgtacca attgctattg taaaaagtgt tgctttcatt gccaagtttg 5940
tttcataaca aaagccttag gcatctccta tggcaggaag aagcggagac agcgacgaag 6000
agctcatcag aacagtcaga ctcatcaagc ttctctatca aagcagtaag tagtacatgt 6060
aacgcaacct ataccaatag tagcaatagt agcattagta gtagcaataa taatagcaat 6120
agttgtgtgg tccatagtaa tcatagaata taggaaaata ttaagacaaa gaaaaataga 6180
caggttaatt gatagactaa tagaaagagc agaagacagt ggcaatgaga gtgaaggaga 6240
aatatcagca cttgtggaga tgggggtgga gatggggcac catgctcctt gggatgttga 6300
tgatctgtag tgctacagaa aaattgtggg tcacagtcta ttatggggta cctgtgtgga 6360
aggaagcaac caccactcta ttttgtgcat cagatgctaa agcatatgat acagaggtac 6420
ataatgtttg ggccacacat gcctgtgtac ccacagaccc caacccacaa gaagtagtat 6480
tggtaaatgt gacagaaaat tttaacatgt ggaaaaatga catggtagaa cagatgcatg 6540
aggatataat cagtttatgg gatcaaagcc taaagccatg tgtaaaatta accccactct 6600
gtgttagttt aaagtgcact gatttgaaga atgatactaa taccaatagt agtagcggga 6660
gaatgataat ggagaaagga gagataaaaa actgctcttt caatatcagc acaagcataa 6720
gaggtaaggt gcagaaagaa tatgcatttt tttataaact tgatataata ccaatagata 6780
atgatactac cagctataag ttgacaagtt gtaacacctc agtcattaca caggcctgtc 6840
caaaggtatc ctttgagcca attcccatac attattgtgc cccggctggt tttgcgattc 6900
taaaatgtaa taataagacg ttcaatggaa caggaccatg tacaaatgtc agcacagtac 6960
aatgtacaca tggaattagg ccagtagtat caactcaact gctgttaaat ggcagtctag 7020
cagaagaaga ggtagtaatt agatctgtca atttcacgga caatgctaaa accataatag 7080
tacagctgaa cacatctgta gaaattaatt gtacaagacc caacaacaat acaagaaaaa 7140
gaatccgtat ccagagagga ccagggagag catttgttac aataggaaaa ataggaaata 7200
tgagacaagc acattgtaac attagtagag caaaatggaa taacacttta aaacagatag 7260

ctagcaaatt aagagaacaa tttggaaata ataaaacaat aatctttaag caatcctcag 7320
gaggggaccc agaaattgta acgcacagtt ttaattgtgg aggggaattt ttctactgta 7380
attcaacaca actgtttaat agtacttggt ttaatagtac ttggagtact gaagggtcaa 7440
ataacactga aggaagtgac acaatcaccc tcccatgcag aataaaacaa attataaaca 7500
tgtggcagaa agtaggaaaa gcaatgtatg cccctcccat cagtggacaa attagatgtt 7560
catcaaatat tacagggctg ctattaacaa gagatggtgg taatagcaac aatgagtccg 7620
agatcttcag acctggagga ggagatatga gggacaattg gagaagtgaa ttatataaat 7680
ataaagtagt aaaaattgaa ccattaggag tagcacccac caaggcaaag agaagagtgg 7740
tgcagagaga aaaaagagca gtgggaatag gagctttgtt ccttgggttc ttgggagcag 7800
caggaagcac tatgggcgca gcctcaatga cgctgacggt acaggccaga caattattgt 7860
ctggtatagt gcagcagcag aacaatttgc tgagggctat tgaggcgcaa cagcatctgt 7920
tgcaactcac agtctggggc atcaagcagc tccaggcaag aatcctggct gtggaaagat 7980
acctaaagga tcaacagctc ctggggattt ggggttgctc tggaaaactc atttgcacca 8040
ctgctgtgcc ttggaatgct agttggagta ataaatctct ggaacagatt tggaatcaca 8100
cgacctggat ggagtgggac agagaaatta acaattacac aagcttaata cactccttaa 8160
ttgaagaatc gcaaaaccag caagaaaaga atgaacaaga attattggaa ttagataaat 8220
gggcaagttt gtggaattgg tttaacataa caaattggct gtggtatata aaattattca 8280
taatgatagt aggaggcttg gtaggtttaa gaatagtttt tgctgtactt tctatagtga 8340
atagagttag gcagggatat tcaccattat cgtttcagac ccacctccca accccgaggg 8400
gacccgacag gcccgaagga atagaagaag aaggtggaga gagagacaga gacagatcca 8460
ttcgattagt gaacggatcc ttggcactta tctgggacga tctgcggagc ctgtgcctct 8520
tcagctacca ccgcttgaga gacttactct tgattgtaac gaggattgtg gaacttctgg 8580
gacgcagggg gtgggaagcc ctcaaatatt ggtggaatct cctacagtat tggagtcagg 8640
aactaaagaa tagtgctgtt agcttgctca atgccacagc catagcagta gctgagggga 8700
cagatagggt tatagaagta gtacaaggag cttgtagagc tattcgccac atacctagaa 8760
gaataagaca gggcttggaa aggattttgc tataagatgg gtggcaagtg gtcaaaaagt 8820
agtgtgattg gatggcctac tgtaagggaa agaatgagac gagctgagcc agcagcagat 8880
agggtgggag cagcatctcg agacctggaa aaacatggag caatcacaag tagcaataca 8940
gcagctacca atgctgcttg tgcctggcta gaagcacaag aggaggagga ggtgggtttt 9000
ccagtcacac ctcaggtacc tttaagacca atgacttaca aggcagctgt agatcttagc 9060
cactttttaa aagaaaaggg gggactggaa gggctaattc actcccaaag aagacaagat 9120
atccttgatc tgtggatcta ccacacacaa ggctacttcc ctgattagca gaactacaca 9180
ccagggccag gggtcagata tccactgacc tttggatggt gctacaagct agtaccagtt 9240
gagccagata agatagaaga ggccaataaa ggagagaaca ccagcttgtt acaccctgtg 9300

agcctgcatg ggatggatga cccggagaga gaagtgttag agtggaggtt tgacagccgc 9360
ctagcatttc atcacgtggc ccgagagctg catccggagt acttcaagaa ctgctgacat 9420
cgagcttgct acaagggact ttccgctggg gactttccag ggaggcgtgg cctgggcggg 9480
actggggagt ggcgagccct cagatcctgc atataagcag ctgctttttg cctgtactgg 9540
gtctctctgg ttagaccaga tctgagcctg ggagctctct ggctaactag ggaacccact 9600
gcttaagcct caataaagct tgccttgagt gcttcaagta gtgtgtgccc gtctgttgtg 9660
tgactctggt aactagagat ccctcagacc cttttagtca gtgtggaaaa tctctagca 9719

## Description

### Field of the invention

The present invention relates to functionalized nucleic acids and to particles comprising such functionalized nucleic acids, which functionalized nucleic acids and particles are useful for treating Human Immunodeficiency Virus (HIV) infections, in particular by inhibiting HIV integrase.

### Background of the invention

Human immunodeficiency virus (HIV) is the causative agent of acquired immunodeficiency syndrome (AIDS). Current treatments, generally referred to as highly active antiretroviral treatments (HAARTs), combine several active drugs which target one or several key steps of the HIV life cycle.

Until recently, drugs that had been formally approved for the treatment of HIV-infected patients belonged to four classes: the nucleoside reverse transcriptase inhibitors (NRTIs), the non-nucleoside reverse transcriptase inhibitors (NNRTIs), the protease inhibitors (PIs), and the fusion inhibitors. However, owing to low-level residual replication and the genetic flexibility of the virus, HIV strains resistant to these drugs have emerged, thereby warranting the search for alternative anti-HIV drugs.

A fifth class of potent anti-HIV drugs, the integrase inhibitors (INIs), which target the third enzyme of HIV, has thus recently been introduced, with the approval of raltegravir (Isentress®) at the end of 2007 in Europe. HIV integrase (IN) is a viral protein that catalyzes the covalent insertion of the viral DNA (vDNA) produced by reverse transcription of HIV RNA genome into the chromosomes of infected cells. Nevertheless, as with the other anti-HIV drugs, resistances have emerged, either *in vitro* or *in vivo*. Accordingly, there is still a need for alternative anti-HIV compounds.

From a biochemical point of view, two spatially and temporally independent reactions catalyzed by the integrase are necessary for the insertion of vDNA into the host cell DNA. First, the viral integrase (IN) binds to a short sequence located at both ends of the vDNA named Long Terminal Repeat (LTR) and catalyzes an endonucleolytic maturation, called 3'-processing, resulting in the removal of a dinucleotide at each end. Then, the trimmed DNA is used as a substrate for strand transfer which leads to the covalent insertion of vDNA into the genome of infected cells. This second catalytic reaction takes place concomitantly on both ends of the vDNA, with a five base pairs interval between the two points of insertion. The formation of the IN·vDNA complex induces a conformational change of IN leading to a highly stable and catalytically slow complex, which does not dissociate after 3'-processing. *In vivo* this property allows the complex to remain associated during the time necessary for translocation and oncoming onto cellular DNA.

In pharmacological terms, two avenues have been considered in the search for inhibitors: either targeting (i) the free, unbound, protein, that is to say before the formation of the complex, or (ii) the complex itself. The feasibility of the two types of inhibition was demonstrated by the identification of both (i) 3'-processing inhibitors that actually impair the binding of the free enzyme to the viral DNA and (ii) strand transfer inhibitors that target the IN·vDNA complex, preventing the latter to bind to the target, namely chromosomal DNA. The stability of the complex and its presence in the cell during the pre-integrative replicative steps make it the most favorable target.

This assumption was confirmed by the remarkable antiviral activity of integrase strand transfer inhibitors (INSTIs), such as raltegravir or elvitegravir, which act by binding to the IN·vDNA complex near the 3' end of donor DNA, thereby selectively blocking the strand transfer step with nanomolar IC₅₀ values *in vitro* and *in vivo*. However, given raltegravir's efficacy, most of the INIs under development aim at having a mechanism of action similar to that of this drug and are therefore likely to be inefficient against raltegravir-resistant HIV strains.

In this regard, INSTIs with different mechanisms of actions have been explored. Thus, oligonucleotide GGTTTTTGTGT (SEQ ID NO: 1) conjugated to acridine was shown to disrupt the IN•vDNA complex with a micromolar IC₅₀ (Pinskaya et al. (2004) Biochemistry 43:8735-8743). In addition, the same olignonucleotide conjugated to eosin presents an excellent IC₅₀ of 50 nM towards IN *in vitro* (Prikazchikova et al. (2007) Mol. Biol. (Moscow) 41:118-125). However, the coupling of eosin to oligonucleotides is not compatible with the conditions of automatic synthesis, which impairs its development in large scales. In addition, the eosin-conjugated oligonucleotide does not cross biological membranes easily, which thus strongly diminishes its *in vivo* efficiency.

Accordingly, there is still a need for alternative INIs to raltegravir likely to efficiently inhibit HIV integration *in vivo*.

### Summary of the invention

The present invention arises from the finding by the present inventors that a particle obtained by the non-covalent association in a 1/10 ratio of (i) an eosinylated 11-mer oligonucleotide and (ii) a cell-penetrating peptide designed for the intracellular delivery of peptides, potently inhibited the cellular replication of Human Immunodeficiency Virus 1 (HIV-1), comprising raltegravir-resistant HIV-1 strains. The present inventors have further evidenced that oligonucleotides functionalized with carboxy-hexachlorofluorescein or with fluorescein and oleic acid present *in vitro* integrase inhibition properties equivalent to that of eosinylated oligonucleotides, while being easier to synthesize.

The present invention thus relates to a particle comprising a functionalized nucleic acid non-covalently associated to a cell-penetrating peptide, wherein:
- the functionalized nucleic acid comprises, or consists of:
   - an oligonucleotide comprising from 11 to 20 contiguous nucleotides; and
   - at least one functionalizing group of the following formula (I) covalently linked to the oligonucleotide: 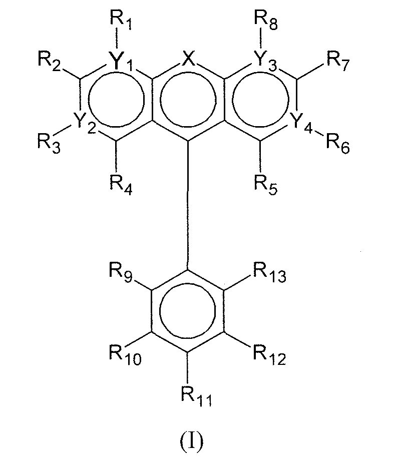 wherein:
      - X, Y₁, Y₂, Y₃ and Y₄, identical or different, represent -C-, -O-, or -N-
      - R₁, R₃, R₆ and R₈, identical or different, represent no group when Y₁, Y₂, Y₃ and Y₄ respectively represent -O-, or -N-, or represent -H, -I, -Br, -Cl, -F, -OH,
      - SH, =O, or -OAlk, wherein Alk represents a methyl, ethyl or propyl group, when Y₁, Y₂, Y₃ and Y₄ respectively represent -C-;
      - R₂, R₄, R₅ and R₇, identical or different, represent -H, -I, -Br, -Cl, -F, -OH, - SH, =O, or -OAlk, wherein Alk represents a methyl, ethyl or propyl group;
      - R₉ and R₁₃, identical or different, represent -H, -I, -Br, -Cl, -F, -OH, -SH, =O,
      - COO- or -OAlk, wherein Alk represents a methyl, ethyl or propyl group;
      - R₁₀, R₁₁ and R₁₂, identical or different, represent -H, -I, -Br, -Cl, -F, -OH, - SH, =O, -NO₂, -OAlk, wherein Alk represents a methyl, ethyl or propyl group, or a linkage with the oligonucleotide, provided one of R₁₀, R₁₁ and R₁₂ represent the linkage with the oligonucleotide;
- the cell-penetrating peptide comprises from 12 to 30 contiguous amino acids and comprises:
   - a first segment, comprising at least 8 amino acids, wherein at least 25% of the amino acids are aromatic amino acids;
   - a second segment, comprising at least 4 contiguous amino acids, wherein at least 75% of the amino acids are basic amino acids;
   - optionally a spacer segment in continuity between the first and second segments comprising at least 1 amino acid;
- the particle is liable to be obtained by contacting the functionalized nucleic acid with the cell-penetrating peptide in a 1/5 to 1/15 molar ratio.

In an embodiment of the above-defined particle, the nucleic acid is further functionalized by at least one fatty acid which is covalently linked to the oligonucleotide.

In another embodiment of the invention, the above-defined particle is for use as a medicament, in particular for use in the treatment of Human Immunodeficiency Virus (HIV) infections and/or Acquired Immune Deficiency Syndrome (AIDS).

The present invention also relates to a first compound comprising a functionalized nucleic acid comprising, or consisting of:
- an oligonucleotide comprising from 11 to 20 contiguous nucleotides; and
- at least one functionalizing group of the following formula (I) covalently linked to the oligonucleotide: 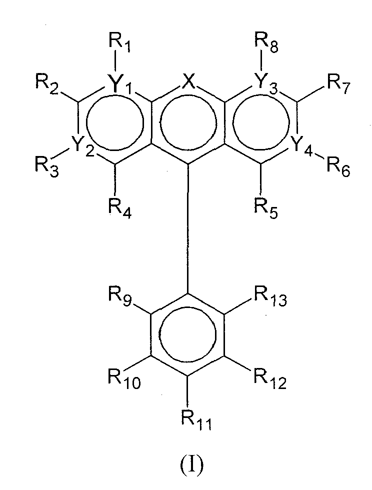 wherein:
   - X, Y₁, Y₂, Y₃ and Y₄, identical or different, represent -C-, -O-, or -N-
   - R₁, R₃, R₆ and R₈, identical or different, represent no group when Y₁, Y₂, Y₃ and Y₄ respectively represent -O-, or -N-, or represent -H, -I, -Br, -Cl, -F, -OH,
   - SH, =O, or -OAlk, wherein Alk represents a methyl, ethyl or propyl group, when Y₁, Y₂, Y₃ and Y₄ respectively represent -C-;
   - R₂, R₄, R₅ and R₇, identical or different, represent -H, -I, -Br, -Cl, -F, -OH, - SH, =O, or -OAlk, wherein Alk represents a methyl, ethyl or propyl group;
   - R₉ and R₁₃, identical or different, represent -H, -I, -Br, -Cl, -F, -OH, -SH, =O,
   - COO⁻ or -OAlk, wherein Alk represents a methyl, ethyl or propyl group;
   - R₁₀, R₁₁ and R₁₂, identical or different, represent -H, -I, -Br, -Cl, -F, -OH, - SH, =O, -NO₂, -OAlk, wherein Alk represents a methyl, ethyl or propyl group, or a linkage with the oligonucleotide, provided one of R₁₀, R₁₁ and R₁₂ represent the linkage with the oligonucleotide; and
- at least one fatty acid covalently linked to the oligonucleotide.

The present invention also relates to a second compound comprising a functionalized nucleic acid comprising, or consisting of:
- an HIV integrase-inhibiting oligonucleotide comprising from 11 to 20 contiguous nucleotides as defined in any of claims 1 to 4; and
- at least one functionalizing group of the following formula (IV) covalently linked to the oligonucleotide: 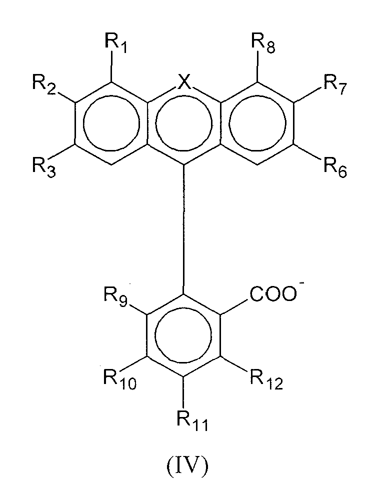 wherein:
   - X represents -O- or -N-;
   - R₁, R₃, R₆, and R₈, identical or different, represent -Br or -Cl;
   - R₂ and R₇ represent =O or -OH;
   - R₉ and R₁₂ represents -H, -Br, or -Cl, provided at least one of R₉ and R₁₂ represents -Br or - Cl.
   - one of R₁₀ and R₁₁ represents -H and the other one represents a linkage with the oligonucleotide.

In an embodiment of the invention, the first compound as defined above and the second compound as defined above are for use as a medicament, in particular for use in the treatment of HIV infections and/or AIDS

The present invention also relates to a pharmaceutical composition comprising at least one particle as defined above, or at least one first or second compound as defined above, as active substance, in association with at least one pharmaceutically acceptable carrier.

The present invention also relates to a method for treating HIV infections or AIDS in an individual, comprising administering the individual with a therapeutically effective amount of at least one particle as defined above, or at least one first or second compound as defined above.

### Detailed description of the invention

### Oligonucleotide

The oligonucleotide of the invention may be made of Deoxyribonucleic Acid (DNA) or of Ribonucleic Acid (RNA). Besides, the backbone of the oligonucleotide may be modified in particular to increase its stability and/or its resistance to hydrolysis or degradation, in particular by nucleases. Such modifications are well known to one of skill in the art. Thus, where the oligonucleotide is made of RNA at least one 2' OH group of the oligonucleotide may be methylated. Preferably, all the 2' OH groups of the oligonucleotide are methylated. In addition, at least one phosphate group of the oligonucleotide may be replaced by a phosphorothioate group. Preferably also, all the phosphate groups of the oligonucleotide are replaced by phosphorothioate groups. Of course, different type of modification may be present in the oligonucleotide of the invention. Thus, the 2' OH groups of the oligonucleotide may be methylated along with the replacement of phosphate groups by phosphorothioate groups. Advantageously, 2' OH methylation and the presence of phosphorothioate groups improve the integrase inhibiting properties of the functionalized nucleic acid of the invention. By way of example, portions of an oligonucleotide of the invention respectively comprising phosphorothioate modifications and methylation of 2' OH groups are represented below:

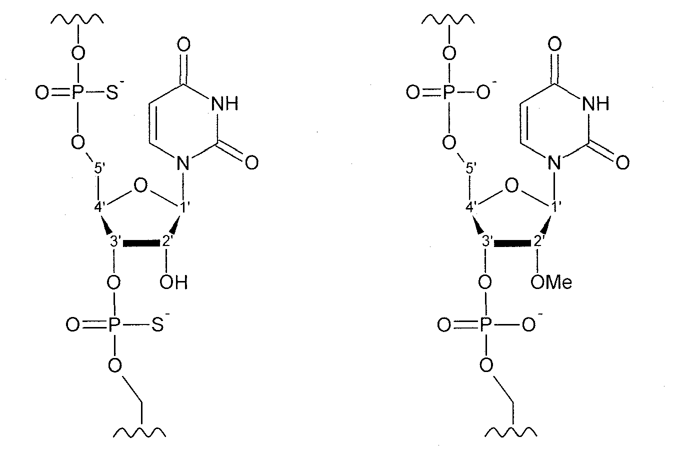

Besides, it has been shown by the inventors that the sequence of the oligonucleotide may be changed with a minimal impact on the integrase inhibiting properties of the functionalized nucleic acid of the invention. Accordingly, the oligonucleotide of the invention may be of any sequence. However, it is preferred that the oligonucleotide of the invention itself presents integrase inhibiting properties. Thus, the oligonucleotide of the invention preferably presents an IC₅₀ (i.e. the concentration of oligonucleotide inhibiting 50% of the enzyme activity) towards the 3'-processing activity of HIV-1 integrase below 20 µM and/or an IC₅₀ towards the strand transfer activity of HIV-1 integrase below 20 µM. The 3'-processing activity and the strand transfer activity of integrase can be determined by numerous methods well known to one of skill in the art and in particular as is described by Leh et al. (2000) Biochemistry 39:9285-9294.

In particular, it is preferred that the oligonucleotide according to the invention comprises, or consists of, a sequence selected from the group consisting of:
- G-G-U/T-U/T-U/T-U/T-U/T-G-U/T-G-U/T (SEQ ID NO: 1);
- U/T-C-U/T-C-A-C-A-A-C-U/T-A (SEQ ID NO: 2); and
- C-U/T-G-A-C-U/T-G-C-A-U/T-C (SEQ ID NO : 3);
- or of a sequence presenting at least 70%, preferably at least 80% identity, and most preferably at least 90% identity with SEQ ID NO: 1, 2 or 3, provided that the oligonucleotide presents an IC₅₀ towards the 3'-processing activity of HIV-1 integrase below 20 µM and/or an IC₅₀ towards the strand transfer activity of HIV-1 integrase below 20 µM.

A sequence presenting at least 70% identity with SEQ ID NO: 1, 2 or 3, is notably derived from SEQ ID NO: 1, 2 or 3 by the insertion, the suppression, or the substitution of at least one nucleotide. As intended herein, the percentage of identity between two sequences is defined as the number of positions for which the bases are identical when the two sequences are optimally aligned, divided by the total number of bases of the longer of the two sequences. Two sequences are said to be optimally aligned when the percentage of identity is maximal. Besides, as will be clear to one of skill in the art, it may be necessary to add gaps in order to obtain an optimal alignment between the two sequences.

As will be clear to one of skill in the art, the expression "U/T" indicates that the nucleotide at this position is either a T, where the oligonucleotide is made of DNA, or U, where the oligonucleotide is made of RNA.

### Fatty acid

The fatty acid of the invention may be saturated or unsaturated. Preferably, the fatty acid of the invention is a mono-unsaturated fatty acid, more preferably an omega-9 mono-unsaturated fatty acid. Preferably also, the fatty acid of the invention comprises from 10 to 30 carbon atoms, more preferably from 15 to 25 carbon atoms and most preferably from 17 to 21 carbon atoms. Most preferably, the fatty acid according to the invention is oleic acid.

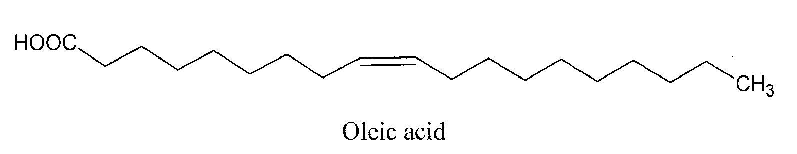

### Cell penetrating peptide

Cell penetrating peptides according to the invention are well-known to one of skill in the art and are notably described in US patent n°US 6,841,535 and in Morris et al. (2004) Gene Therapy 11:757-764 for delivering peptides into cells.

Preferably, the cell-penetrating peptide comprises from 18 to 24 contiguous amino acids. Preferably also, the aromatic amino acids from the first segment are selected from Y, F, and W, in particular from F and W. Still preferably the basic amino acids of the second segment are selected from K and R.

It is particularly preferred that the cell-penetrating peptide according to the invention comprises, or consists of, the following sequence:
- KETWFETWFTEWSQPKKKRKV (SEQ ID NO: 4);
- or a sequence presenting at least 70%, preferably at least 80% identity, and most preferably at least 90% identity with SEQ ID NO: 4, provided that a particle obtained by associating, preferably in water at 37°C and at pH 7, a 3' eosinylated oligonucleotide of sequence SEQ ID NO: 1 with the cell penetrating peptide, which is preferably at a concentration of from 0.5 to 2 mM, in a 1/10 molar ratio, presents an EC₅₀ (*i.e.* the concentration of the particle inhibiting 50% of a virus infectivity) for HIV-1, in particular towards HeLa-P4 cells, lower than 1 µM.

A sequence presenting at least 70% identity with SEQ ID NO: 4, is notably derived from SEQ ID NO: 4 by the insertion, the suppression, or the substitution of at least one amino acid. As intended herein, the percentage of identity between two sequences is defined as the number of positions for which the amino acids are identical when the two sequences are optimally aligned, divided by the total number of bases of the longer of the two sequences. Two sequences are said to be optimally aligned when the percentage of identity is maximal. Besides, as will be clear to one of skill in the art, it may be necessary to add gaps in order to obtain an optimal alignment between the two sequences.

Besides, the cell penetrating peptide of the invention may comprise modifications at the N-terminus and/or at the C-terminus. Thus, the cell penetrating peptide of the invention may be N-acetylated (Ac) at the N-terminus and/or bear a cysteamide group (Cya, of formula
- NH-CH₂-CH₂-SH) at the C-terminus. Thus most preferably, the cell-penetrating peptide according to the invention consists of the following sequence:
   - Ac-KETWFETWFTEWSQPKKKRKV-Cya (SEQ ID NO: 4).

### Particle

The particles of the invention are liable to be obtained by contacting the functionalized nucleic acid with the cell penetrating peptide in a 1/5 to 1/15, preferably 1/9 to 1/11, and more preferably 1/10 molar ratio.

As intended herein, in a x/y molar ration x represents the quantity or the concentration of the functionalized nucleic acid and y represents the quantity or the concentration of the cell penetrating peptide. According to the invention, the functionalized nucleic acid and the cell penetrating peptide are preferably contacted in water, preferably at pH 7, preferably at 37°C, and preferably with a concentration of the cell penetrating peptide from 0.5 to 2 mM. The particles of the invention generally have a diameter of from 50 nm to 150 nm. Accordingly, the particles of the invention are preferably nanoparticles. Without wishing to be bound to a particular theory, it is believed that the cell penetrating peptides auto-assemble so as to encage the functionalized nucleic acids.

In a preferred embodiment of the particle according to the invention, the functionalizing group has the following formula (II): 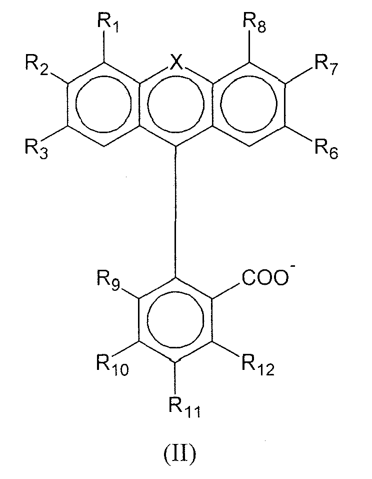 wherein:
- X represents -O- or -N-;
- R₁, R₃, R₆, and R₈, identical or different, represent -Br or -Cl;
- R₂ and R₇ represent =O or -OH;
- R₉ and R₁₂ represents -H, -Br, or -Cl;
- one of R₁₀ and R₁₁ represents -H and the other one represents a linkage with the oligonucleotide.

In another preferred embodiment of the particle according to the invention, the functionalizing group has the following formulae (III) or (V): 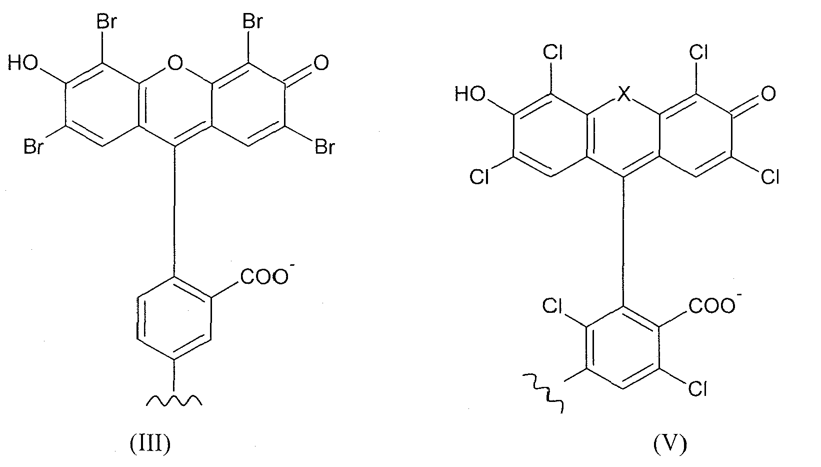 wherein X represents -O- or -N-.

In a particularly preferred embodiment of the particle according to the invention:
- the functionalized nucleic acid consists of a RNA oligonucleotide, in particular wherein all the 2' OH groups are methylated, consisting of the sequence G-G-U-U-U-U-U-G-U-G-U (SEQ ID NO: 1) or U-C-U-C-A-C-A-A-C-U-A (SEQ ID NO: 2), wherein the oligonucleotide is linked at its 3' extremity by a functionalizing group having the following formula (III): 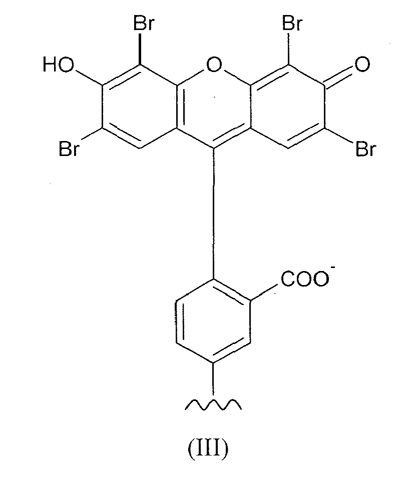
- the cell-penetrating peptide consists of the sequence Ac-KETWFETWFTEWSQPKKKRKV-Cya (SEQ ID NO: 4);
- the particle is obtained by contacting the functionalized nucleic acid with the cell-penetrating peptide in a 1/10 molar ratio.

### First and second compounds

In a preferred embodiment of the first compound, the functionalizing group has the following formula (II): 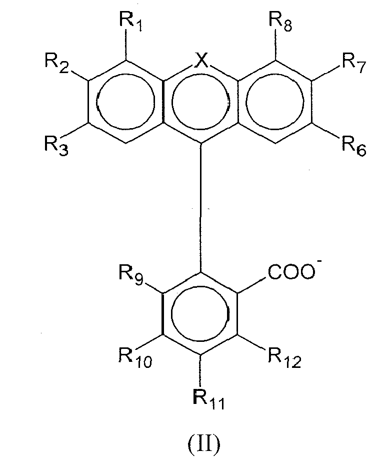 wherein:
- X represents -O- or -N-;
- R₁, R₃, R₆, and R₈, identical or different, represent -Br or -Cl;
- R₂ and R₇ represent =O or -OH;
- R₉ and R₁₂ represents -H, -Br, or -Cl;
- one of R₁₀ and R₁₁ represents -H and the other one represents a linkage with the oligonucleotide.

In another preferred embodiment of the first compound, the functionalizing group has the following formula formula (VI):

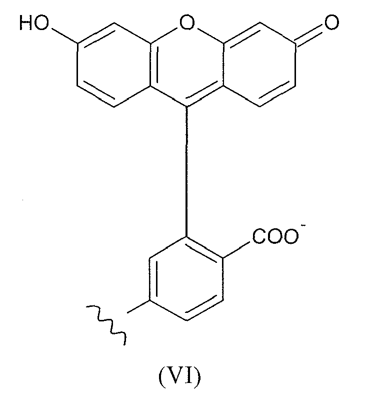

The compound of formula (I) is also known as fluorescein.

In yet another preferred embodiment of the first compound, the functionalized nucleic acid consists of a DNA oligonucleotide consisting of the sequence G-G-T-T-T-T-T-G-T-G-T (SEQ ID NO: 1) linked at its 3' extremity by oleic acid and at its 5' or at its 3'extremity by a functionalizing group having the formula (VI).

In a preferred embodiment of the second compound, the functionalizing group has the following formula (V): 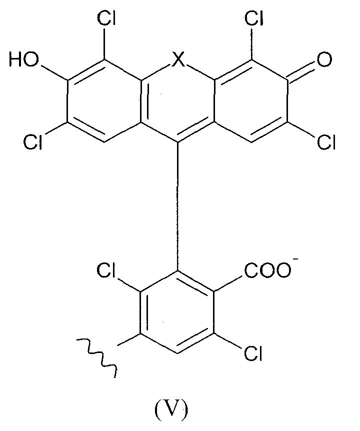 wherein X represents -O- or -N-.

In a preferred embodiment of the second compound, the functionalized nucleic acid consists of a RNA oligonucleotide, in particular wherein all the 2' OH groups are methylated, consisting of the sequence G-G-U-U-U-U-U-G-U-G-U (SEQ ID NO: 1) linked at its 5' extremity by a functionalizing group having the formula (V).

### Linking

In accordance with the invention, the functionalizing groups of the invention may be covalently linked to any part of the oligonucleotide of the invention. However, it is preferred that the functionalizing groups are linked to the 5' and/or 3' extremities, or ends, of the oligonucleotide according to the invention. As will clear to one of skill in the art, where several functionalizing groups are linked to the oligonucleotide of the invention, for instance where both a functionalizing group of formula (I) and a fatty acid are linked to the oligonucleotide, the groups may be all linked to the 3' end of the oligonucleotide, all linked to the 5' end of the oligonucleotide, or at the 5' end and at the 3' end of the oligonucleotide.

Besides, numerous methods are known to one of skill in the art, in particular versed in the field of fluorescently labeled nucleic acids, for covalently linking a functionalizing group, such as a group of formula (I) to an oligonucleotide. Any such method can be used in accordance with the invention. In this regard, as will be clear to one of skill in the art, the functionalizing group may be directly or indirectly linked, through a linking group, to the oligonucleotide. Such linking groups, which are covalently linked both to the oligonucleotide and to the functionalizing group, are well known to one skilled in the art. A preferred linking group according to the invention comprises from 1 to 100 carbon atoms. Preferred linking group according to the invention are selected from the group consisting of:

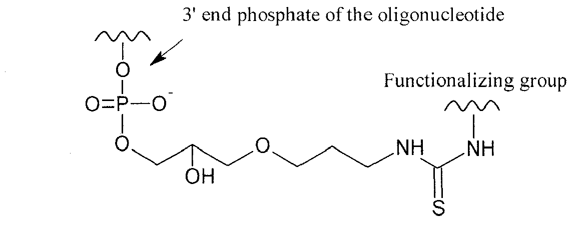

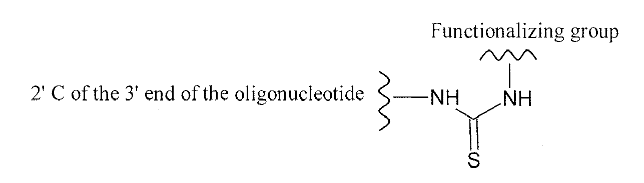

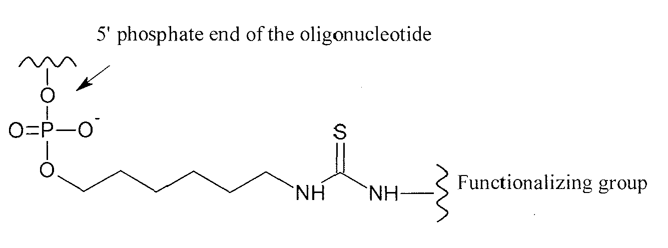

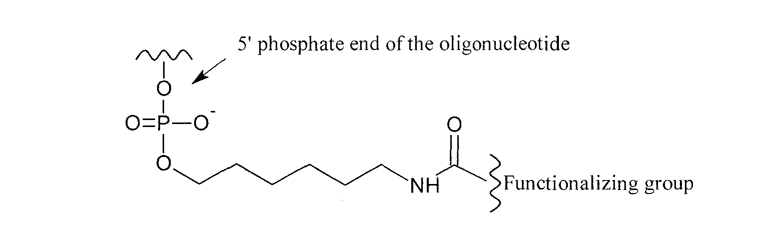

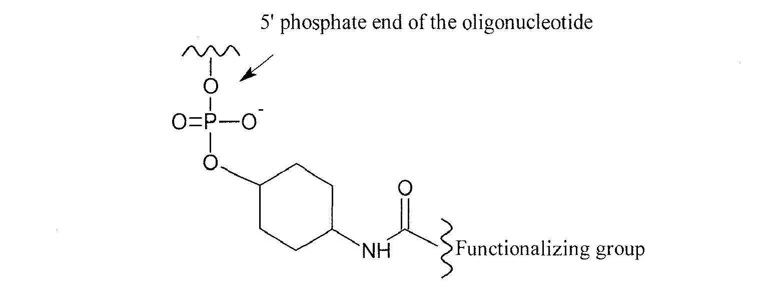

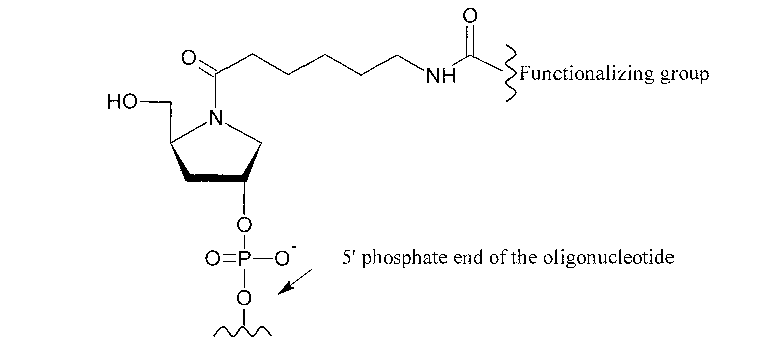

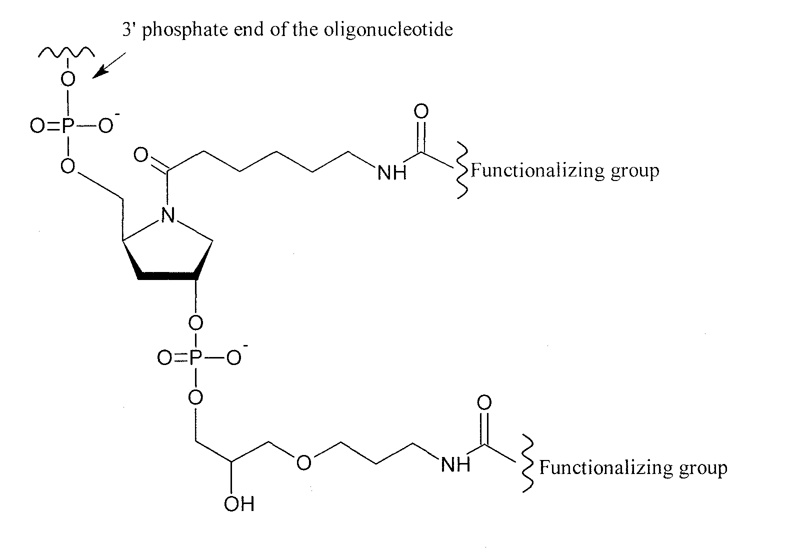

As will be clear to one of skill in the art, in the above preferred representations of linking group where the functionalizing group is a fatty acid and the functionalizing group is linked to the linking group through a carbonyl group, the carbonyl group is preferably that of the carboxyl moiety of the fatty acid.

### Nucleic acid

Preferably, the functionalized nucleic acid according to the invention consists of one oligonucleotide according to the invention to which are covalently linked one or more functionalizing groups according to the invention.

### Medicament, Pharmaceutical composition

The particles, compounds, medicaments and pharmaceutical compositions according to the invention are useful for treating HIV, in particular HIV-1, infections and/or AIDS, in particular by inhibiting HIV, notably HIV-1, integrase.

The particles according to the invention should preferably be administered with a dosage of from 0,1 mg/kg to 50 mg/kg, more preferably of from 1 mg/kg to 20mg/kg. The preferred administration route of the particles of the invention is the intravenous route or the topical route.

The particles and compounds of the invention may be administered with pharmaceutically acceptable carriers, vehicles or excipients. As used herein, the expression "pharmaceutically acceptable" refers to those carriers, vehicles or excipients which are, within the scope of sound medical judgment, suitable for contact with the tissues of human beings and animals without excessive toxicity, irritation, allergic response, or other problem complications commensurate with a reasonable benefit/risk ratio.

In addition, the particles, compounds, medicaments and pharmaceutical compositions according to the invention may be administered in association with one or several other drugs, in particular anti-HIV drugs, notably NRTIs, NNRTIs, PIs, fusion inhibitors, or INIs, in particular INSTIs, such as raltegravir or elvitegravir.

The invention will now be further described by the following non-limiting figures and examples.

### Description of the figures

Figure 1

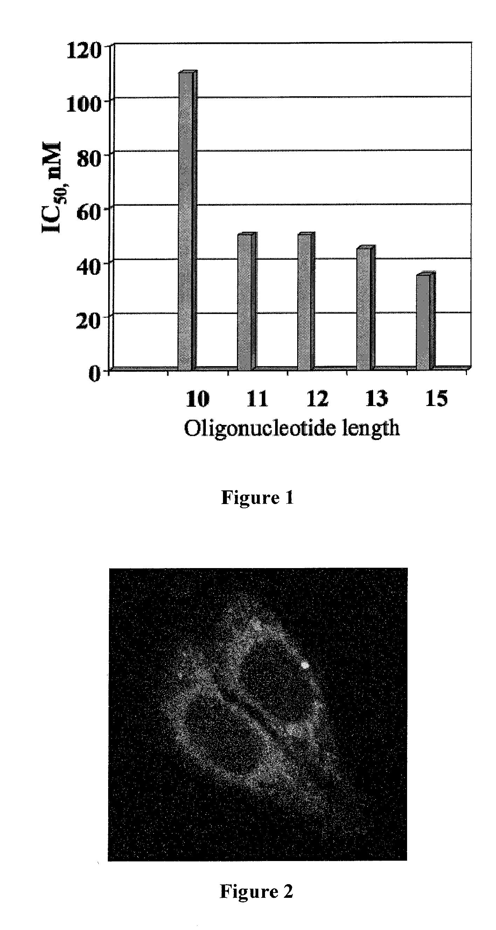

   Figure 1 represents the IC₅₀ (vertical axis, nM) of GG(T)n oligonucleotides linked to eosin on HIV-1 integrase activity as a function of oligonucleotide length (horizontal axis, n = 10, 11, 12, 13, 15)
Figure 2
   Figure 2 represents a fluorescent microscopy image of cells contacted by particle 310 obtained by mixing an eosinylated oligonucleotide with a Pep-2 cell penetrating peptide in a 1/10 ratio.
Figure 3

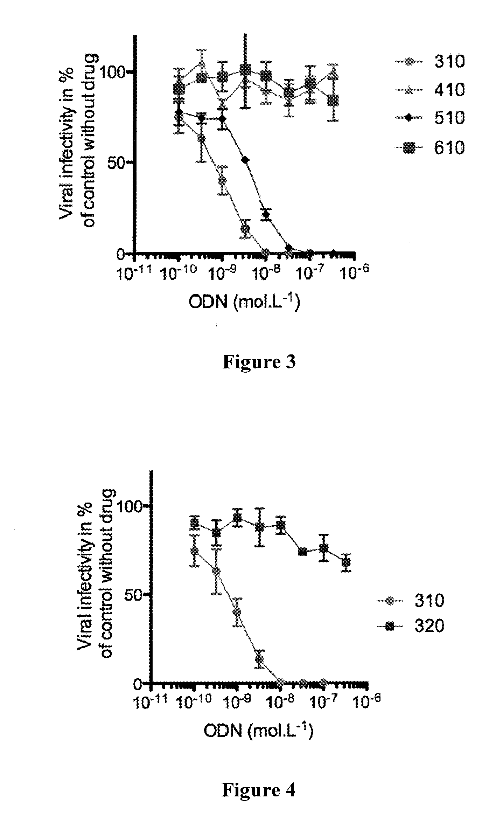

   Figure 3 represents the viral infectivity of HIV-1 towards HeLa-P4 cells (vertical axis, percentage of infectivity of control without drug) in the presence of increasing concentrations of the oligonucleotide (ODN) functionalized or not (horizontal axis, in mol.L⁻¹) comprised in particles 310 (circles), 410 (triangles), 510 (diamonds) and 610 (squares).
Figure 4
   Figure 4 represents the viral infectivity of HIV-1 towards HeLa-P4 cells (vertical axis, percentage of infectivity of control without drug) in the presence of increasing concentrations of the oligonucleotide (ODN) functionalized or not (horizontal axis, in mol.L⁻¹) comprised in particles 310 (circles) and 420 (squares).
Figure 5

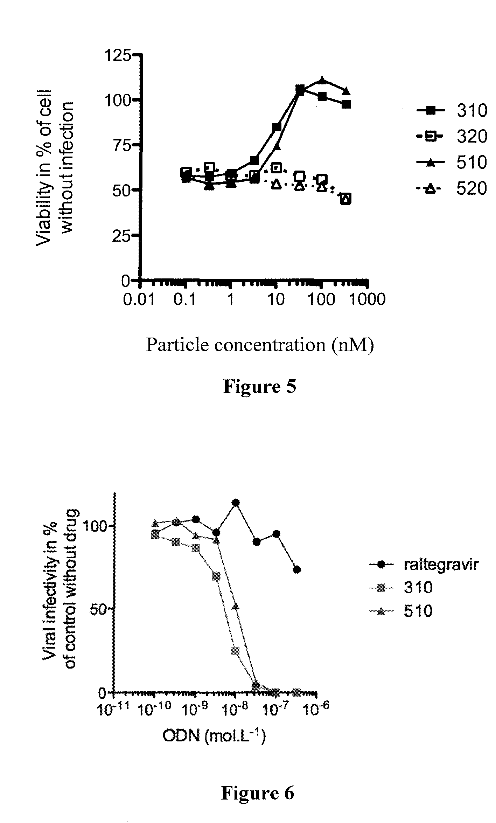

   Figure 5 represents the viability of CEM T4 cells infected with HIV-1 expressed as a percentage of the viability uninfected cells (vertical axis) in the presence of increasing concentrations (horizontal axis, nM) of the 310 (black squares), 320 (white squares), 510 (black triangles) and 520 (white triangles) particles.
Figure 6
   Figure 6 represents the viral infectivity of a HIV-1 raltegravir-resistant strain towards HeLa-P4 cells (vertical axis, percentage of infectivity of control without drug) in the presence of increasing concentrations of the eosinylated oligonucleotide (ODN) (horizontal axis, mol.L⁻¹) comprised in particles 310 (full), and 510 (triangles) and of raltegravir (circles).
Figure 7

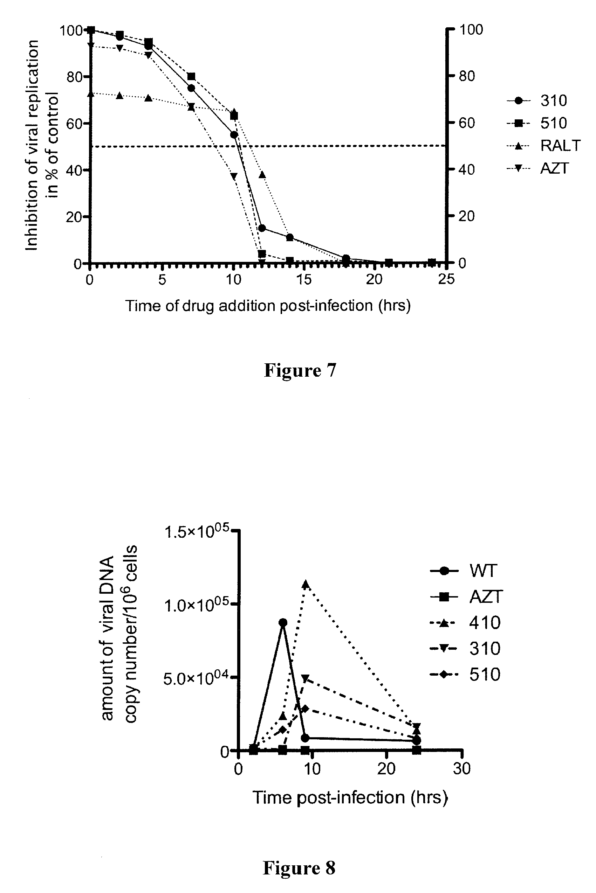

   Figure 7 represents the inhibition of viral replication (vertical axis, percentage of replication of control without drug) due to a drug selected from particle 310 (100 nM) (circles), particle 510 (100 nM) (square), raltegravir (100 nM) (RALT, triangles), and AZT (1 µM) (inverted triangles), as function of the time of drug addition post-infection (horizontal axis, hours).
Figure 8
   Figure 8 represents the amount of total viral DNA in infected cells infected by HIV-1 (vertical axis, number of copies per cell) determined by Q-PCR in the absence of any drug (infection control, WT, circles) or in the presence of particle 310 (100 nM) (inverted triangles), particle 410 (100 nM) (triangles), particle 510 (100 nM) (diamonds), and AZT (1 µM) (squares) as function of the time post-infection (horizontal axis, hours).

### Examples

### Example 1

In this Example, the inventors carried out step-by-step modifications of both the oligonucleotide and the functionalizing group of the functionalized nucleic acid of the invention and tested the influence of these modifications on the inhibitory effect. This structure-activity relationship analysis was aimed at the determination of the functionalized nucleic acid structural features responsible for integrase (IN) inhibition in order to elucidate the mechanism of inhibitor binding to IN. Besides, this study has allowed the inventors to optimize the structure of the inhibitor and methods for its synthesis.

### 1. Materials and methods

### 1.1. Protein expression and purification

Detergent-free recombinant HIV-1 IN from HXB2 viral strain (GenBank Accession Number K03455, SEQ ID NO: 7) was produced in *Escherichia coli* and purified as described by Leh et al. (2000) Biochemistry 39:9285-9294.

### 1.2. Functionalized oligonucleotide synthesis

All oligonucleotides were synthesized by the phosphoramidite method on an automatic ABI 3400 DNA synthesizer (Applied Biosystems, USA), under conditions recommended by manufacturer, and purified by electrophoresis in a 20% polyacrylamide/7 M urea gel.

Modified oligonucleotides were additionally purified by RP-HPLC with an acetonitrile gradient in ammonium acetate (pH 7).

5'-FAM, FAM, TET, HEX phosphoramidites were purchased from Metkinen Chemistry Oy (Finland); dT, dA(Bz), dG(iBu), dC(Ac), ddR, 1,3-propanediol phosphoramidites and dT p-methyl phosphonamidite - from ChemGenes (USA).

Oligonucleotides with eosin and oleic acid at 3'-end were synthesized as described by Prikazchikova et al. (2007) Mol. Biol. (Moscow) 41:130-138. Phosphorothioate oligonucleotides were synthesized as described by Krotz et al. (2004) Org. Proc. Res. Dev. 8:852-858. Oligonucleotides with methylphosphonate linkages were synthesized as described by Arnold et al. (1993) Methods Mol Biol. 20:143-64.

### 1.3. 3'-Processing activity assay

A DNA duplex consisting of oligonucleotides U5B (5'-GTGTGGAAAATCTCTAGCAGT-3', SEQ ID NO: 5) and U5A (5'-ACTGCTAGAGATTTTCACAC-3', SEQ ID NO: 6), and mimicking the end of the HIV-1 U5 LTR was used as an IN substrate. The U5B oligonucleotide (10 pmol) was labeled with T4 polynucleotide kinase (Fermentas, Lithuania) and 50 µCi [γ-³²P]ATP (3000 Ci/mmol). After 1 h of incubation at 37°C, the T4 polynucleotide kinase was inactivated by adding EDTA and heating at 65°C for 5 minutes. The U5B oligonucleotide was then annealed with an equimolar amount of the complementary oligonucleotide, U5A. The resulting U5B/U5A duplex was then purified from unincorporated [γ-³²P]ATP by centrifugation through MicroSpin G-25 Columns (GE Healthcare, UK). The ³²P-labeled substrate, U5B/U5A (3 nM), was incubated in 20 µl of 20 mM Hepes, pH 7.2, 7.5 mM MgCl₂, 1 mM DTT with 100 nM IN, in the presence of increasing concentrations of an oligonucleotide inhibitor (0.01-10 µM) at 37°C for 2 h. The reaction was stopped by adding 80 µl of a stop solution (7 mM EDTA, 0.3 M sodium acetate, 10 mM Tris-HCl, pH 8). IN was extracted in phenol/chloroform, and DNA fragments were precipitated with ethanol. The reaction products were suspended in loading dye (80% formamide, 0.05% bromophenol blue, 0.05% xylene cyanol) and separated by electrophoresis in a 20% polyacrylamide/7 M urea gel. Gel images were recorded on a STORM 840TM Phosphorlmager (Molecular Dynamics, USA) and quantified with Image QuaNTTM 4.1 software (USA).

### 2. Results and discussion

### 2.1. Inhibitory potency of conjugates with different mode of functionalization of the oligonucleotide

The inventors have previously shown that the inhibitory activity of the oligonucleotide functionalized with an hydrophobic moiety does not depend on the location of the functionalizing group at the 3'- or 5'-end (Prikazchikova et al. (2007) Mol. Biol. (Moscow) 41:130-138). Nevertheless, to choose an optimal method for the functionalizing group attachment to oligonucleotide 11D (GGTTTTTGTGT, SEQ ID NO: 1), the inventors varied eosin (E) and fluoresceine (Fl) linking and then studied the dependence of the inhibitory potency on the structure of linkers between 11D and the fluorophores. E was attached to the 3'-end of 11 D either through a flexible linker (11D-E) or directly to a 2'-amino group in the terminal nucleoside (11D-E2). Fl was attached to the 3'-end phosphate similarly to E (11D-Fl) or to the 5'-end through a rigid hydroxyprolinol moiety (FAM-11D).

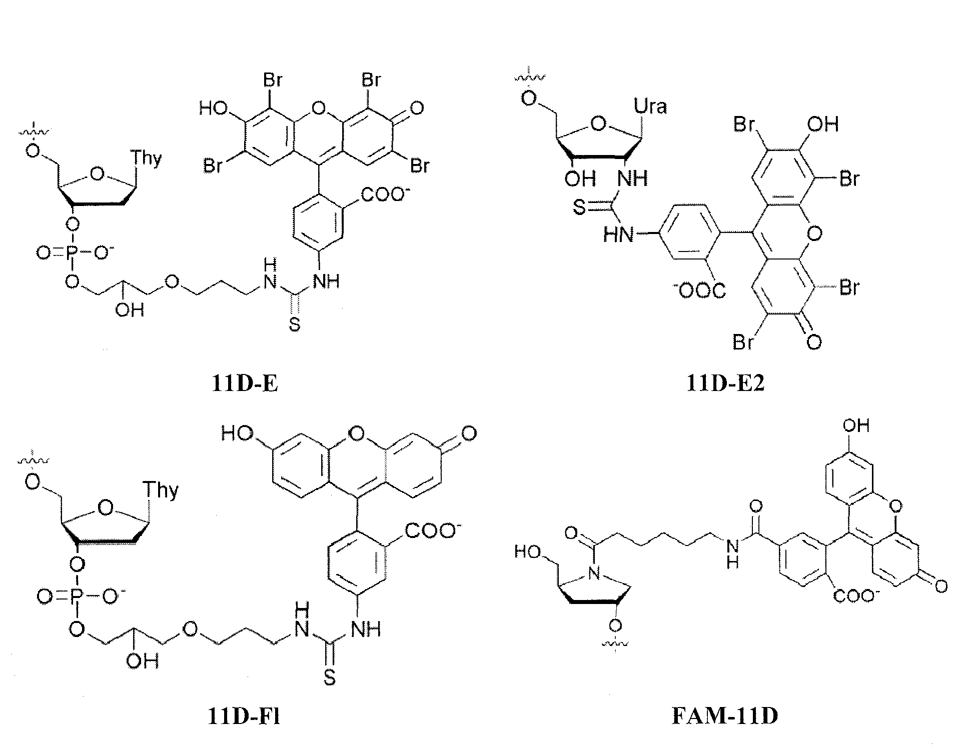

As previously shown in Pinskaya et al. (2004) Biochemistry 43:8735-8743, the binding of functionalized nucleic acids to the IN•vDNA complex and its subsequent disruption leads to a strong inhibition of the 3'-processing (3'-P). Thus the potency of the functionalized nucleic acids was monitored by quantifying their capability to inhibit this reaction. Conjugates containing the same functionalizing group showed comparable inhibitory potency; IC₅₀ (concentration of the inhibitor inhibiting 50% of integrase activity) was about 50 nM for 11D-E and 11D-E2 and 300 nM for 11D-Fl and FAM-11D. Therefore, the structure of the link between the functionalizing group and the oligonucleotide does not alter the capability of the functionalized nucleic acids to inhibit HIV-1 IN.

### 2.2. Synthesis of oligodeoxynucleotides functionalized by different functionalizing groups

The synthesis of the most efficient inhibitor 11D-E is rather laborious, thus thwarting its further development for pharmacological use. Indeed, E is destroyed during post-synthetic deprotection of oligonucleotides under basic conditions, and as a result it cannot be coupled to oligonucleotides during the automatic synthesis. Therefore, an additional step of E coupling to 3'-amino containing deprotected oligonucleotides by using eosin-iso-thiocyanate followed by electrophoresis or HPLC purification is necessary for the conjugate preparation.

In order to avoid this additional step, the inventors replaced E by a set of hydrophobic molecules that can be easily attached to oligonucleotides during the automatic synthesis, and then tested the capacity of these novel conjugates to inhibit IN.

Since the link between the functionalizing group and 11D does not affect anti-integrase activity, the inventors used corresponding 5'-modifying agents to simplify the synthesis of the functionalized nucleic acid. Thus all the molecules were attached at the 5'-end of 11D. The structure-activity relationships (SAR) in these new compounds were investigated by coupling fluorescein derivatives with different substituents such as 6-carboxyfluorescein (FAM), 6-carboxy-4,7,2',7'-tetrachlorofluorescein (TET), 6-carboxy-4,7,2',4',5',7'-hexachlorofluorescein (HEX), and 2,4,5,7-tetrachloro-3,6-dihydroxy-9-(2',5'-dichloro-4',6'-dicarboxyphenyl)acridin (HEXAcr) to 11D (**Table 1**).

Besides, conjugates containing FAM and oleic acid (Ole) at either one or both ends of 11D were synthesized by using CPG support with attached Ole and a phosphoramidite ((2S,4R)-N-(6-(3',6'-dipivaloylfluoresceinyl-6-carboxamido)hexanoylamido)-4-O-[(2-cyanoethyl)-(N,Ndiisopropyl)-phosphoramidite]-2-(dimethoxytrityloxymethyl)pyrrolidine) that could be incorporated inside the oligonucleotide chain.

### 2. 2. Effect of the hydrophobic moiety structure on the inhibitory activity of the conjugates

All these functionalized 11D were tested for their ability to inhibit the catalytic activity of recombinant IN in 3'-P and compared to 11D-E (**Table 1**).

**Table 1. Effect of the functionalizing group structure on 3'-P inhibition**

| **Name** | **Structure of the functionalizing group** | **Attachement site** | **3'-P IC₅₀ (nM)** |
|---|---|---|---|
| 11D-E | 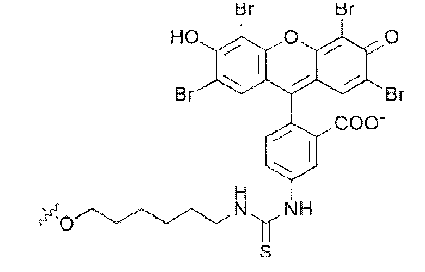 | 3' end | 50 ± 7 |
| FAM-11D | 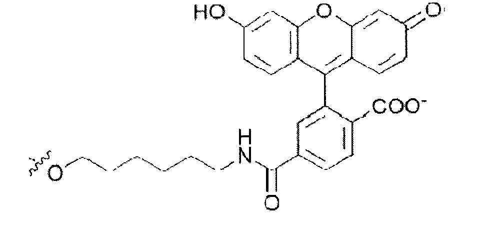 | 5' end | 320 ± 50 |
| HEX-11D | 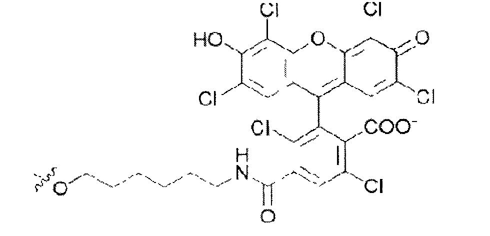 | 5' end | 55 ± 10 |
| HEX_{Acr}-11D | 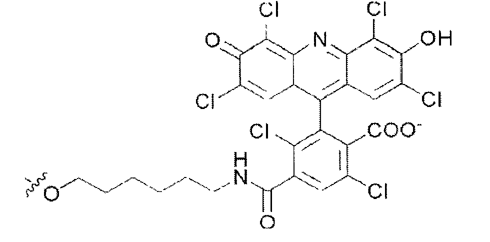 | 5' end | 52 ± 8 |
| TET-11D | 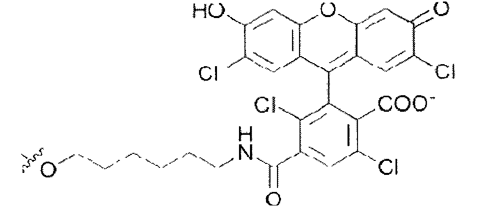 | 5' end | 290 ± 40 |
| JOE-11D | 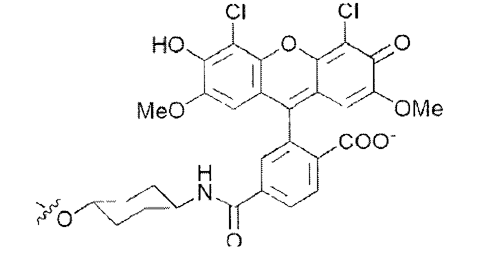 | 5' end | 310 ± 35 |
| 11D-Ole | 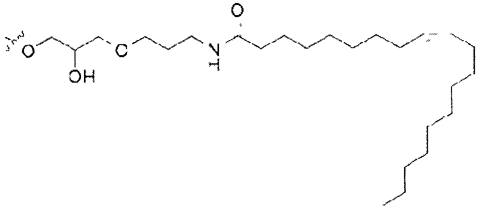 | 3' end | 200 ± 25 |
| FAM-11D-Ole | 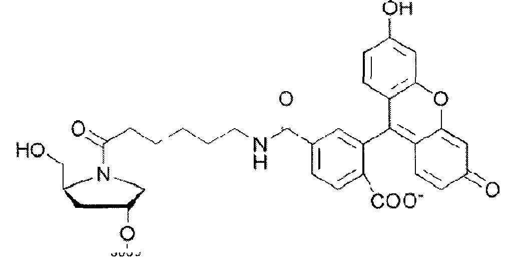 | 5' end | 20 ± 5 |
| | 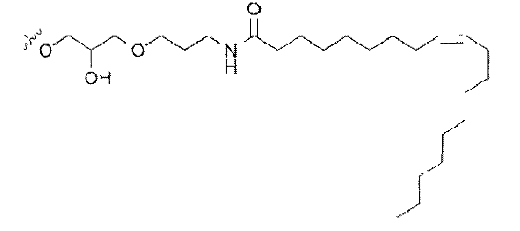 | 3' end | |
| 11 D-(FAM)Ole | 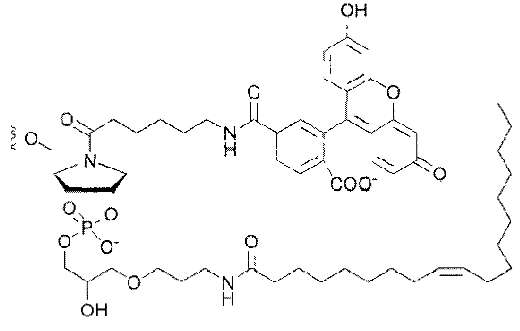 | 3' end | 23 ± 4 |

Complete substitution of bromine by chlorine on the triple ring fragment had no influence on the conjugate inhibitory activity. Indeed, conjugates with HEX and HEXAcr showed the same inhibitory potency as the 11D-E compound. A change in the triple ring fragment resulting from the oxygen substitution by nitrogen (HEX-11D → HEXAcr-11D) did not affect the inhibitory activity. Compounds containing TET and JOE showed a 6-fold decrease in 3'-P inhibition as compared to 11D-E and demonstrated the same ability to inhibit IN as FAM-11D. Therefore, the presence of two chlorine atoms is not sufficient to improve the inhibitory potency. Moreover, it may be concluded that the location of chlorine atoms has no influence on the IN inhibition, only the number of halogen atoms on the triple ring fragment is important.

Continuing SAR studies of the hydrophobic groups, the inventors studied conjugates of the oligonucleotide 11D with two hydrophobic molecules. The simultaneous presence of FAM and Ole in the conjugates significantly improved their inhibitory potency when compared to the mono-substituted compounds (**Table 1**, compare compounds FAM-11D-Ole and 11 D-(FAM)Ole with FAM-11D and 11D-Ole). This effect, which did not depend on FAM location, might be due to the enhanced hydrophobicity of the bi-substituted compounds.

### 2.3. Influence of the oligonucleotide length and structure on the inhibitory activity of the conjugates

It has been previously shown that the shortening of the oligonucleotide part in Fl-functionalized nucleic acids reduces their inhibitory potency (Prikazchikova et al. (2007) Mol. Biol. (Moscow) 41:130-138).

Accordingly, the inventors synthesized oligodeoxynucleotides GG(T)n, where n=8-13 functionalized with E, and tested their inhibitory effect. A conjugate of the 10-mer turned out to be twice less efficient than the 11-mer compound whereas conjugates of longer oligonucleotides showed about the same level of 3'-P inhibition (**Figure 1**).

It can therefore be concluded that 11-mer oligonucleotides have the optimal length for the conjugate preparation. Decrease of the conjugate inhibitory potency resulting from oligonucleotide shortening as well as a weak effect of the oligonucleotide sequence on the inhibitory activity (Prikazchikova et al. (2007) Mol. Biol. (Moscow) 41:130-138) indicate that the main role of the oligonucleotide moiety relies on the electrostatic contacts with Lys and Arg residues of IN.

To check this assumption, the inventors changed the oligonucleotide charge without altering its length by replacing phosphodiester groups with methylphosphonates. Three conjugates were synthesized containing three methylphosphonates at different positions: near 3'-end, near 5'-end and uniformly distributed along the oligonucleotide chain (**Table 2**, 11DX-E). All the conjugates inhibited IN approximately two-fold less efficiently than 11D-E. Thus, the removal of three negative charges produced the same effect as the removal of one nucleotide bearing one negative charge (**Figure 1**). This indicates that the total amount of nucleotides, but not only the charge, is important for the inhibitory activity. Moreover, the same activity of conjugates 11DX-E-1, 11DX-E-2 and 11DX-E-3 shows that the negative charge distribution is not important for interactions with IN.

Furthermore, the inventors replaced all or several phosphodiester groups with phosphorothioates and found that this modification improved inhibitory activity (**Table 2**). This could be explained by both the increased localization of the negative charge on sulfur atoms when compared to oxygen atoms and higher hydrophobic properties of phosphorothiates than those of phosphodiesters.

Similarly methylphosphonate modification, localization of phosphorothioates along the oligonucleotide chain had no effect on IN inhibition. Moreover, partial or total phosphorothioate incorporation resulted in the same IC₅₀ values (**Table 2**).

In view of the important influence of oligonucleotide shortening on the inhibitory potency of the functionalized nucleic acid, the inventors continued the SAR studies of the oligonucleotide structure and determined the role of heterocyclic bases. Thus, a series of HEX conjugates containing 1,2-dideoxyribose (ddR) or 1,3-propanediol (PD) instead of 10 or 6 native nucleosides was synthesized (**Table 2**). These compounds had the same number of phosphates as HEX-11D, and in the case of ddR modification the conjugates retained the same length and structure of the sugar-phosphate backbone. PD-containing compounds have a flexible structure. The absence of heterocyclic bases resulted in a significant decrease of the inhibitory activity of the conjugates (**Table 2**). The substitution of six nucleotides by both ddR and PD resulted in a 5 to 6-fold increase of IC₅₀ whereas the absence of 10 bases led to a 24-fold increase of IC₅₀. Similarly to modifications of the internucleotide phosphates described above, there was no correlation between the anti-integrase activity and localization of the modified residues in the oligonucleotide; compounds HEX-11D-ddR-1 and HEX-11D-ddR-2 as well as HEX-11D-PD-1 and HEX-11D-PD-2 demonstrated approximately the same effect (**Table 2**).

Thus, the presence of heterocyclic bases is important for the efficient inhibition of IN activity. They might be involved in interactions with positively charged amino acids, which are favored by the base accessibility in the single-stranded oligonucleotide. Interestingly, Arg and Lys may be involved in contacts with all the nucleotide bases (Lejeune et al. (2005) PROTEINS: Structure, Function, and Bioinformatics 61:258-271), and this fact can explain why the IN inhibition by functionalized oligonucleotide is not sequence-specific.

**Table 2. Effect of oligonucleotide structure modification on 3'-P inhibition**

| **Name** | **Structure of the modification** | **Oligonucleotide 5' → 3'** | **3'-P IC₅₀ (nM)** |
|---|---|---|---|
| 11D-E | - | GGTTTTTGTGT-**E** | 50 ± 7 |
| 11DX-E-1 | 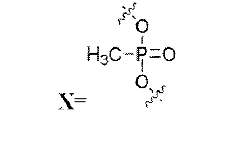 | GGTTTTT**x**GT**x**GT**x**-**E** | 100 ± 15 |
| 11DX-E-2 | | GGT**x**T**x**T**x**TTGTGT-**E** | 110 ± 20 |
| 11DX-E-3 | | GGT**x**TT**x**TTGT**x**GT-**E** | 100 ± 15 |
| 11DS-E-1 | 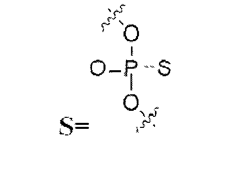 | G**s**G**s**T**s**T**s**T**s**T**s**T**s**G**s**T**s**G**s**T**s**-**E** | 18 ± 4 |
| 11DS-E-2 | | GGTTTTT**s**GT**s**GT**s**-**E** | 20 ± 2 |
| 11DS-E-3 | | GGT**s**T**s**T**s**TTGTGT-**E** | 22 ± 6 |
| 11DS-E-4 | | GGT**s**TTT**s**TGT**s**GT-**E** | 24 ± 7 |
| HEX-11D | 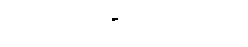 | **HEX**-GGTTTTTGTGT | 55 ± 10 |
| HEX-11-ddR | 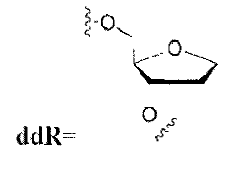 | **HEX**-(**ddR**)₁₀T | 1200 ± 70 |
| HEX-11D-ddR-1 | | **HEX**-(**ddR**)₆TGTGT | 280 ± 35 |
| HEX-11D-ddR-2 | | **HEX**-GGTT(**ddR**)₆T | 250 ± 30 |
| HEX-11-PD | 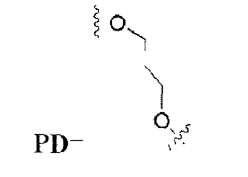 | **HEX**-(**PD**)₁₀T | 1300 ± 90 |
| HEX-11D-PD-1 | | **HEX**-(**PD**)₆TGTGT | 250 ± 25 |
| HEX-11D-PD-2 | | **HEX**-GGTT(**PD**)₆T | 310 ± 45 |

### 2.4. Inhibitory activity of conjugates with multiple modifications

In view of the improved potency of the phosphorothioate-containing and bi-functionalized nucleic acids, the inventors further synthesized a 2'-O-methyl-oligonucleotide of sequence GGUUUUUGUGU (11-OM, SEQ ID NO: 1) containing full-length phosphorothioate internucleotide linkages (11MS) functionalized with HEX at the 5'-end (HEX-11MS) and another one additionally containing 3'-end Ole (HEX-11MSOle). These oligonucleotides were 2'-O-methylated in order to increase their resistance to nucleases in subsequent cellular experiments. Surprisingly, inhibitory activity of both compounds was comparable (3'-P IC₅₀ equal to 20 nM). Thus, attachment of Ole to the efficient inhibitor HEX-11MS did not further improve its potency whereas its attachment to the average inhibitor FAM-11D (IC₅₀ about 300 nM) provided more than 10-fold enhancement of 3'-P inhibitory activity (**Table 2**, compound FAM-11D-Ole).

Similarly, replacement of all internucleotide phosphodiesters with phosphorothioates (compound 11DS-E-1) did not additionally increase the inhibitory potency when compared to the conjugates containing only 3 phosphorothioates (**Table 2**). It should be also noted that IC₅₀ for the best inhibitors was not less than 20 nM. Taking into account that at least IN dimer is necessary to accomplish 3'-P (Faure et al. (2005) Nucleic Acids Research 33:977-986) and that the IN concentration used was about 50 nM, we can assume that concentration of the catalytically active IN dimmers did not exceed 20-25 nM and thus corresponds to IC₅₀ for the best inhibitors. It can therefore be hypothesized that, in order to be active, the conjugate must bind all IN subunits to prevent their interactions with the substrate DNA.

In conclusion, the above SAR studies of oligonucleotide conjugates with hydrophobic molecules indicate that:
(1) eosin can be replaced by HEX, since HEX containing functionalized nucleic acids show the same anti-IN activity while their synthesis is easier;
(2) phosphorothioate oligonucleotides are favored for the conjugate preparation due to their enhanced inhibitory potency;
(3) both internucleotide phosphates and nucleic bases are important;
(4) additional attachment of Ole to HEX-containing phosphorothioates does not enhance the inhibitory activity but might be useful to improve their penetration into cells.

### Example 2

In this example the inventors have manufactured a particle formed by the association of an eosinylated 2'-O-methyl-oligonucleotide with a cell penetrating peptide. This particle shows nanomolar activity against the replication of both wild type and INSTIs-resistant HIV-1 virus in cell culture.

### 1. In vitro HIV-1 integrase inhibition

2'-O-methyl-oligonucleotides 11-OM (GGUUUUUGUGU, SEQ ID NO: 1) and 11-OM2 (UCUCACAACUA, SEQ ID NO: 2) were functionalized with eosin as described above for 11D-E2 to yield 11-OM-E and 11-OM2-E.

The *in vitro* inhibition efficiencies of HIV-1 integrase (3' processing and strand transfer) by the various oligonucleotides were then determined according to Leh et al. (2000) Biochemistry 39:9285-9294 and are reported in **Table 3**.

**Table 3. Anti-integrase efficiency of functionalized oligonucleotides**

| EOSINYLATED OLIGONUCLEOTIDES | | | IC₅₀, µM | |
|---|---|---|---|---|
| Oligonucleotide sequence | Eosin | Functionalized oligonucleotide | 3'-processing | Strand transfer |
| GGUUUUUGUGU | - | 11-OM | 11 ± 2 | 10 ± 3 |
| | + | 11-OM-E | 0.05 ± 0.02 | 0.06 ± 0.03 |
| UCUCACAACUA | - | 11-OM2 | 13 ± 1 | 12 ± 3 |
| | + | 11-OM2-E | 0.08 ± 0.02 | 0.09 ± 0.03 |

It can be seen that the particles have inhibitory efficiencies *in vitro* similar to that of the eosin-functionalized oligonucleotides they comprise.

### 2. Intracellular delivery of the functionalized oligonucleotide by the particles

The oligonucleotides were then associated with the Pep-2 cell penetrating peptide (Ac-KETWFETWFTEWSQPKKKRKV-Cya, SEQ ID NO: 4) at 1/10 and 1/20 molar ratios as indicated in **Table 4**. A peptide similar to the Pep-2 cell-penetrating peptide was described in Morris et al. (2004) Gene Therapy 11:757-764 for introducting short peptides and peptide analogues of nucleic acids (Peptide Nucleic Acids, PNAs) into cells.

The oligonucleotide/peptide complexes, *i.e*. the particles, were obtained by directly adding a peptide stock solution of Pep-2 to the oligonucleotides in water or in DMEM at 37°C. The Pep-2 stock solution is prepared in water (GIBCO ultrapure water, pH 7.0), at concentration between 0.5 and 2 mM.

**Table 4. definition of the particles**

| EOSINYLATED OLIGONUCLEOTIDES | | | PARTICLE (OLIGONUCLEOTIDE/PEP-2 RATIO) |
|---|---|---|---|
| Oligonucleotide sequence | Eosin | Functionalized oligonucleotide | Particle Name |
| GGUUUUUGUGU | - | 11-OM | 410 (1/10) |
| | | | 420 (1/20) |
| | + | 11-OM-E | 310 (1/10) |
| | | | 320 (1/20) |
| UCUCACAACUA | - | 11-OM2 | 610 (1/10) |
| | | | 620 (1/20) |
| | + | 11-OM2-E | 510 (1/10) |
| | | | 520 (1/20) |

In order to determine the most active nanoparticle composition, several functionalized oligonucleotide/Pep-2 ratios were tested for cellular internalization.

The eosinylated-oligonucleotide cell delivery was evaluated by monitoring the intrinsic fluorescence of the functionalized oligonucleotides. Briefly, adherent fibroblastic Hela cells were cultured in Dulbecco's Modified Eagle's Medium supplemented with 2mM glutamine, 1% antibiotics (streptomycin 10'000 µg/ml, penicillin, 10'000 IU/ ml) and 10% (w/v) foetal calf serum (FCS), at 37°C in a humidified atmosphere containing 5% CO₂. Oligonucleotide/Pep-2 complexes were formed by incubation of oligonucleotide with Pep-2 at a molecular ratio of 1/20 or 1/10, respectively, in 500 µl of DMEM for 30 min at 37 °C. Cells grown to 60 % confluence were then overlaid with these preformed complexes. For cellular localization experiments, cells were grown on acid-treated glass coverslips to 60% confluence then overlaid with preformed oligonucleotide/Pep-2 complexes. After 6 hours, cells were rinsed twice and cellular localization of intrinsic fluorescence of the oligonucleotide was monitored by fluorescence microscopy on living cells.

For all ratios tested, a fast and efficient intracellular internalization was observed. The preferred intracellular distribution varied depending upon the ratio. Interestingly, nanoparticles formed with a 1/10 ratio essentially yielded a cytoplasmic delivery of the oligonucleotide (see **Figure 2**).

### 3. Antiviral activity of the particles

The particles were first tested in a single-round replication assay in Hela-P4 cells. The HeLa-P4 cells are described by Delelis et al. (2010) Nucleic Acid Res. 54:491-501. These cells are HeLa CD4 LTR-LacZ cells in which lacZ expression is induced by HIV transactivation due to the Tat protein, making it possible to quantify HIV-1 infectivity from a single cycle of replication.

Briefly, cells were infected, in triplicate, in 96-well plates, with virus (3 ng of p24 gag antigen). Viral infectivity was determined 48 hours after infection by quantifying beta-galactosidase activity in P4 lysates by a colorimetric assay based on the cleavage of chlorophenol red-beta-D-galactopyranoside by beta-galactosidase (CPRG assay). For IC50 determination, cells were infected with viruses during 2 hours.Cells were then washed in Phosphate Buffer Saline (PBS) and grown in the presence of increasing concentrations of particles. The 50% inhibitory concentration (IC50) was determined as the particles concentration giving 50% inhibition of beta-galactosidase levels with respect to untreated infected cells.

As shown in **Figure 3**, particles 310 and 510 showed a remarkable antiviral activity in the single-round infection assay with EC₅₀ values respectively in the range of 10⁻⁹ and 10⁻⁸ mol/l, *i.e*. comparable to that of reference compounds raltegravir and elvitegravir. In contrast, control particles 410 and 610 lacking eosin were inactive. Besides, particles formed either without functionalized oligonucleotides, on one hand, or without the cell-penetrating peptide, on the other hand, possessed essentially no antiviral activity.

In addition, **Figure 4** shows that when both the 1/10 and 1/20 ratios were tested, only the 1/10 ratio was active, thus demonstrating the importance of the nature of the particle. This result also pointed out that the antiviral activity of the functionalized oligonucleotide might be related to its cytoplasmic localization.

The 310, 320, 510 and 520 particles were further tested in a multiple-round of infection cytoprotection assay of CEM T4 cells infected with pNL4.3 HIV-1 clone.

Briefly, CEM-T4 cells were infected with pNL4.3 at a multiplicity of infection of 0.1 for 2 hours. The virus was removed by extensive washing with phosphate-buffer and increasing concentration of the particles (0.1 nM up to 333 nM) were added to the cell culture-medium. Cell survival was determined by a standard MTT (3-[4,5-dimethylthiazol-2-yl]-2,5- -diphenyltetrazolium bromide) assay 6 days post-infection.

**Figure 5** shows that the active particles 310 and 510 protected the cells against the cytopathogenicity induced by viral replication.

Besides, the cytotoxicity of both active particles 310 and 510 was evaluated by growing Hela-P4 or CEM-T4 cell lines in the presence of the particles for 5 days. No significant toxicity was observed up to the highest concentration tested (1 µM).

Eventually, the active particles 310 and 510 were tested against the replication of a viral strain resistant to raltegravir (**Figure 6**) in the single-round replication assay of the pNL4.3 virus which codes an integrase with the G140S/Q148H mutations.

The G140S/Q148H mutant strain replicated efficiently in the presence of high concentrations of raltegravir, thereby confirming the resistance to raltegravir conferred by these mutations. Conversely, the antiviral efficiency of the 310 and 510 particles was not altered, indicating that the G140S/Q148H mutant remained sensitive to these compounds.

### 4. Determining the viral target by time-of-addition experiment

In order to determine the replication step blocked by the antiviral particles of the invention, the particles were added to Hela P4 cell cultures at various times post-infection with pNL4.3 virus and the resulting antiviral activity was compared in the single-round infection assay to that of a standard RTI (AZT) and a standard INI (raltegravir). Antiviral activity was quantified using the above-described CPRG assay. The results of this experiment are shown in **Figure 7**.

The activity of the 310 and 510 particles was maximized for a time of addition post-infection between 0 and 5 hr. The activity decreased gradually and continuously until 12 hours post-infection. This window time of activity is intermediate between those observed with AZT and raltegravir. It is comparable to the activity observed for the integrase inhibitors that causes a destabilization of the early pre-integration complex (Bonnenfant et al. (2004) J. Virol. 78:5728-5736). Moreover, in this model, raltegravir did not block 100% of the viral replication whatever the time of addition post-infection. This effect is characteristic of the mode of action of this inhibitor that blocks strand transfer integration while provoking the accumulation of complete viral circular forms allowing a basal expression of β-galactosidase. By comparison, the particles 310 and 510 completely inhibited the expression of the reporter gene, indicating that inhibition affected an early replicative step, which resulted in a complete absence of full-length viral genomes.

To confirm this hypothesis, viral genomes were quantified by quantitative PCR following infection in the presence of 100 nM of active (310, 510) or inactive (410) particles.

Briefly, 293T cells were infected with Δenv pNL4-3 virus in the presence of 100nM nanoparticles (either the active nanoparticles 310 and 510 or the inactive nanoparticles 410) or 1 µM of the reverse transciptase inhibitor AZT. At different time post-infection, DNA from 5x10⁶ cells was extracted with the DNA blood mini kit (Qiagen) according to the manufacturer instructions. Viral DNA was then analysed by real-time PCR as previously described in (Brussel et al., 2005, Methods Mol Biol, 304, 139-154). All results were reported for 10⁶ cells.

As shown in **Figure 8**, the active nanoparticles displayed a significant effect on the full amount of newly synthesized DNA present in infected cells 12 hr post-infection. This observation confirmed an early effect of the particles on the formation of stable viral genomes.

Altogether, these observations suggest that the particles block the formation of a stable and integration-competent pre-integration complex. This antiviral effect is consistent with the anti-integrase *in vitro* activity of functionalized oligonucleotides, which destabilize the integrase/vDNA complex. It is also in agreement with the cytoplasmic localization of the functionalized oligonucleotide upon delivery by particles obtained with a 1/10 ratio.

In conclusion, the inventors have designed and synthesized eosinylated-oligonucleotides which display an HIV-1 antiviral activity comparable to nanomolar inhibitors of HIV-1. Furthermore, while the IC50 *in vitro* are in the range of those obtained for raltegravir in similar experimental conditions (IC50 = 10 nM), these derivatives present the major advantage of remaining active against integrase mutants, such as the raltegravir-resistant G140S/Q148H mutant.

## Claims

1. A particle comprising a functionalized nucleic acid non-covalently associated to a cell-penetrating peptide, wherein:
• the functionalized nucleic acid comprises, or consists of:
- an oligonucleotide comprising from 11 to 20 contiguous nucleotides; and
- at least one functionalizing group of the following formula (I) covalently linked to the oligonucleotide: 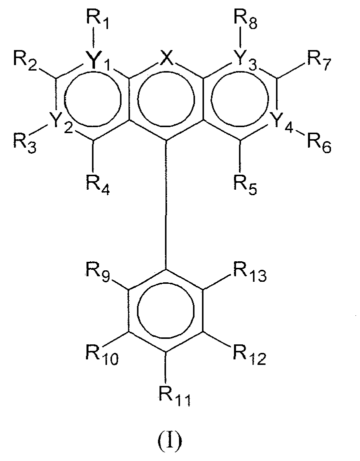 wherein:
- X, Y₁, Y₂, Y₃ and Y₄, identical or different, represent -C-, -O-, or -N-
- R₁, R₃, R₆ and R₈, identical or different, represent no group when Y₁, Y₂, Y₃ and Y₄ respectively represent -O-, or -N-, or represent -H, -I, -Br, -Cl, -F, -OH,
- SH, =O, or -OAlk, wherein Alk represents a methyl, ethyl or propyl group, when Y₁, Y₂, Y₃ and Y₄ respectively represent -C-;
- R₂, R₄, R₅ and R₇, identical or different, represent -H, -I, -Br, -Cl, -F, -OH, - SH, =O, or -OAlk, wherein Alk represents a methyl, ethyl or propyl group;
- R₉ and R₁₃, identical or different, represent -H, -I, -Br, -Cl, -F, -OH, -SH, =O,
- COO⁻ or -OAlk, wherein Alk represents a methyl, ethyl or propyl group;
- R₁₀, R₁₁ and R₁₂, identical or different, represent -H, -I, -Br, -Cl, -F, -OH, - SH, =O, -NO₂, -OAlk, wherein Alk represents a methyl, ethyl or propyl group, or a linkage with the oligonucleotide, provided one of R₁₀, R₁₁ and R₁₂ represent the linkage with the oligonucleotide;
• the cell-penetrating peptide comprises from 12 to 30 contiguous amino acids and comprises:
- a first segment, comprising at least 8 amino acids, wherein at least 25% of the amino acids are aromatic amino acids;
- a second segment, comprising at least 4 contiguous amino acids, wherein at least 75% of the amino acids are basic amino acids;
- optionally a spacer segment in continuity between the first and second segments comprising at least 1 amino acid;
• the particle is liable to be obtained by contacting the functionalized nucleic acid with the cell-penetrating peptide in a 1/5 to 1/15 molar ratio.

2. The particle according to claim 1, wherein the oligonucleotide comprises, or consists of, a sequence selected from the group consisting of:
- G-G-U/T-U/T-U/T-U/T-U/T-G-U/T-G-U/T (SEQ ID NO: 1);
- U/T-C-U/T-C-A-C-A-A-C-U/T-A (SEQ ID NO: 2); and
- C-U/T-G-A-C-U/T-G-C-A-U/T-C (SEQ ID NO : 3).

3. The particle according to claim 1 or 2, wherein the oligonucleotide is Ribonucleic Acid (RNA).

4. The particle according to claim 3, wherein at least one 2' OH group of the oligonucleotide is methylated and/or at least one phosphate group of the oligonucleotide is a phosphorothioate group.

5. The particle according to any of claims 1 to 4, wherein the functionalizing group has the following formula (II): 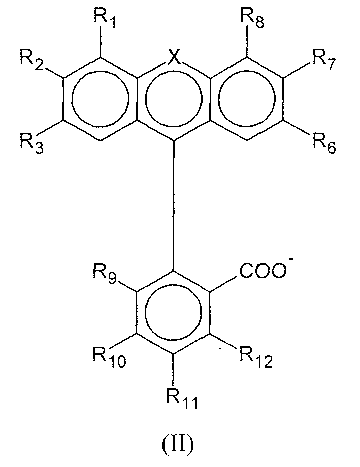 wherein:
- X represents -O- or -N-;
- R₁, R₃, R₆, and R₈, identical or different, represent -Br or -Cl;
- R₂ and R₇ represent =O or -OH;
- R₉ and R₁₂ represents -H, -Br, or -Cl;
- one of R₁₀ and R₁₁ represents -H and the other one represents a linkage with the oligonucleotide.

6. The particle according to any of claims 1 to 5, wherein the cell-penetrating peptide comprises, or consists of, the following sequence:
- KETWFETWFTEWSQPKKKRKV (SEQ ID NO: 4).

7. The particle according to any of claims 1 to 6, wherein the particle is liable to be obtained by contacting the functionalized nucleic acid with the cell penetrating peptide in a 1/9 to 1/11 molar ratio.

8. The particle according to any of claims 1 to 7, wherein:
- the functionalized nucleic acid consists of a RNA oligonucleotide consisting of the sequence G-G-U-U-U-U-U-G-U-G-U (SEQ ID NO: 1) or U-C-U-C-A-C-A-A-C-U-A (SEQ ID NO: 2), wherein the oligonucleotide is linked at its 3' extremity by a functionalizing group having the following formula (III): 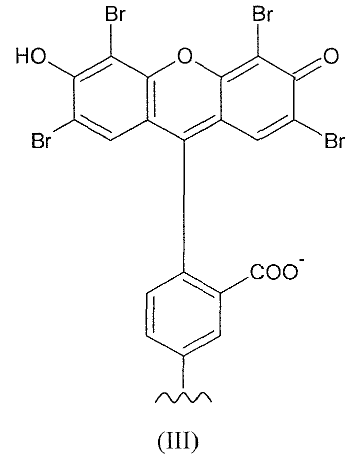
- the cell-penetrating peptide consists of the sequence Ac-KETWFETWFTEWSQPKKKRKV-Cya (SEQ ID NO: 4);
- the particle is obtained by contacting the functionalized nucleic acid with the cell-penetrating peptide in a 1/10 molar ratio.

9. The particle according to any of claims 1 to 8, for use as a medicament.

10. The particle according to any of claims 1 to 9, for use in the treatment of HIV infections.

11. A compound comprising a functionalized nucleic acid comprising, or consisting of:
- an HIV integrase-inhibiting oligonucleotide comprising from 11 to 20 contiguous nucleotides as defined in any of claims 1 to 4; and
- at least one functionalizing group of the following formula (IV) covalently linked to the oligonucleotide: 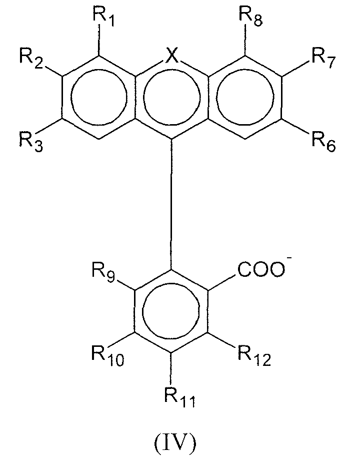 wherein:
- X represents -O- or -N-;
- R₁, R₃, R₆, and R₈, identical or different, represent -Br or -Cl;
- R₂ and R₇ represent =O or -OH;
- R₉ and R₁₂ represents -H, -Br, or -Cl, provided at least one of R₉ and R₁₂ represents -Br or - Cl.
- one of R₁₀ and R₁₁ represents -H and the other one represents a linkage with the oligonucleotide.

12. The compound of claim 11, wherein the functionalized nucleic acid consists of a RNA oligonucleotide consisting of the sequence G-G-U-U-U-U-U-G-U-G-U (SEQ ID NO: 1) linked at its 5' extremity by a functionalizing group having the following formula (V): 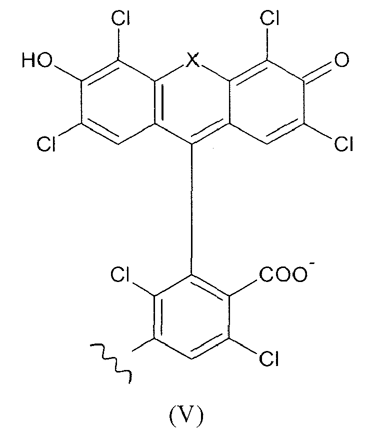 wherein X represents -O- or -N-.

13. The compound according to claim 11 or 12, for use as a medicament

14. The compound according to claim 11 or 12, for use in the treatment of HIV infections.

15. A pharmaceutical composition comprising at least one particle as defined in any of claims 1 to 8, or at least one compound as defined in claim 11 or 12, as active substance, in association with at least one pharmaceutically acceptable carrier.
